(19) Europäisches Patentamt
European Patent Office
Office européen des brevets

(11) **EP 2 960 648 B1**

(12) **EUROPEAN PATENT SPECIFICATION**

(45) Date of publication and mention
of the grant of the patent:
**29.03.2017 Bulletin 2017/13**

(51) Int Cl.:
*G01N 30/34* (2006.01)    *B01D 15/16* (2006.01)
*G01N 33/49* (2006.01)    *G01N 33/72* (2006.01)
*G01N 30/26* (2006.01)    *G01N 30/88* (2006.01)

(21) Application number: **15173715.2**

(22) Date of filing: **24.06.2015**

(54) **MEASUREMENT METHOD, MEASUREMENT APPARATUS, AND ELUENT FOR THE ANALYSIS OF HEMOGLOBINS**

MESSVERFAHREN, MESSVORRICHTUNG UND ELUENT ZUR ANALYSE VON HAEMOGLOBIN

PROCEDE DE MESURE, APPAREIL DE MESURE ET ELUANT POUR L'ANALYSE D'HÉMOGLOBINE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **26.06.2014 JP 2014131769**
**17.06.2015 JP 2015122201**

(43) Date of publication of application:
**30.12.2015 Bulletin 2015/53**

(73) Proprietor: **ARKRAY, Inc.**
**Minami-ku**
**Kyoto-shi**
**Kyoto 601-8045 (JP)**

(72) Inventors:
• **ISHIKAWA, Kazuki**
  **Kyoto-shi, Kyoto 602-0008 (JP)**
• **SAKAI, Toshikatsu**
  **Kyoto-shi, Kyoto 602-0008 (JP)**

(74) Representative: **Golding, Louise Ann**
**Dehns**
**St Bride's House**
**10 Salisbury Square**
**London EC4Y 8JD (GB)**

(56) References cited:
**EP-A1- 2 562 539**      **US-A1- 2009 071 233**

**Description**

BACKGROUND

Technical Field

The present invention relates to a measurement method and a measurement apparatus which measure various kinds of hemoglobin variants, and an eluent used in measuring various kinds of hemoglobin variants.

Related Art

Hemoglobin variants, among them, glycosylated hemoglobin (HbAlc) of glycated protein in which hemoglobin is bound to glucose reflects the average blood glucose level over the past one to two months and thus is widely used for the inspection, glycemic control and the like of lifestyle-related diseases including diabetes and metabolic syndrome. Hence, a method of measuring a hemoglobin variant such as HbA1c easily and with high accuracy is desired.

Examples of the method of measuring HbA1c include a high-performance liquid chromatography (HPLC) method, an immunoassay method, an enzymatic method and an electrophoresis method. Among these, the HPLC method has been often adopted in the field of clinical laboratory test as a standard measurement method of HbA1c.

Various methods are mentioned as the method of separating and measuring hemoglobin variants using the HPLC method. For example, a method of analyzing hemoglobin variants is disclosed in Japanese Patent Application Laid-Open (JP-A) No. H05-281222 in which a liquid containing S-(carboxyalkyl)-L-cysteine and a phosphoric acid-based buffer substance is supplied to a separation column in the method of analyzing hemoglobin variants in a sample by supplying a phosphoric acid-based buffer solution as the eluent to the separation column having a carboxyl group or a carboxyalkyl group as an ion exchange group. According to the technique of JP-ANo. H05-281222, it is regarded that the hemoglobin variants such as HbA1c, HbF, and HbA0 can be separated.

In addition, a method of measuring hemoglobin variants is disclosed in JP-ANo. 2009-236768 in which a buffer solution containing a polysaccharide is used as a buffer solution. According to the technique of JP-A No. 2009-236768, it is regarded that abnormal hemoglobin (HbS, HbC) can be measured and separated from HbA1c, HbF, and HbA2. Furthermore, in Patent Document 2, high-performance liquid chromatography is adopted as the principle and a gradient method is used in which the measurement apparatus used for measuring the abnormal hemoglobin has two supply pumps to supply the eluent to a column, one supply pump is connected to a tank containing one eluent, and the eluent is supplied to the column while changing the mixing ratio of two kinds of eluents.

EP 2 562 539 A1 describes a method for analyzing hemoglobins by liquid chromatography which includes pre-treating a sample with an oxidant and a buffer for trivalent heme iron. Amongst the eluents which may be used is phosphoric acid.

US 2009/0071233 A1 describes a method for analyzing the concentration of glue in an electrolyte solution using high-performance liquid chromatography.

SUMMARY OF THE INVENTION

It is not possible to achieve the separation of hemoglobin variants other than HbA1c, HbF, and HbA0 in the technique of JP-A No. H05-281222, and the separation of hemoglobin variants other than HbA1c, HbF, and HbA2 is limited to HbS and HbC in the technique of JP-A No. 2009-236768. Hence, other than the prior arts described above, a measurement method and a measurement apparatus for measuring various kinds of hemoglobin variants with high accuracy, and an eluent used in measuring various kinds of hemoglobin variants with high accuracy are desired.

An object of the invention is to provide a measurement method, a measurement apparatus and an eluent that can measure at least one kind of hemoglobin selected from abnormal hemoglobin or hemoglobin that is a marker for thalassemia. The invention relates to a measurement method according to claim 1, said method being for measuring at least one kind of hemoglobin selected from abnormal hemoglobin or hemoglobin that is a marker for thalassemia. The invention further relates to a measurement apparatus for measuring a hemoglobin variant by high-performance liquid chromatography according to claim 8.

Specific means for achieving the above object are as follows.

<1> An aspect of the invention is a method of measuring at least one kind of hemoglobin, selected from abnormal hemoglobin, or hemoglobin that is a marker for thalassemia, the method including: performing high-performance liquid chromatography, using: an eluent L that contains a phosphoric acid monohydrogen dialkali metal salt (component 1) and a phosphoric acid dihydrogen monoalkali metal salt (component 2) as components, and that has a content of component 1 of from 0.08% by mass to 0.50% by mass with respect to a total mass of the eluent L, a content of component 2 of from 0.04% by mass to 1.2% by mass with respect to a total mass of the eluent L, and a ratio of component 2/component 1 of from 0.4 to 10.

<2> An aspect of the invention is a method of measuring at least one kind of hemoglobin, selected from abnormal hemoglobin, or hemoglobin that is a marker for thalassemia, the method including: performing high-performance liquid chromatography, using: an eluent O that contains a phosphoric acid monohydrogen dialkali metal salt (component 1) and a phosphoric acid dihydrogen monoalkali metal salt (component 2) as components, and that has a content of component 1 of less than 0.07% by mass with respect to a total mass of the eluent O, a content of component 2 of from more than 1.4% by mass to 2.0% by mass with respect to a total mass of the eluent O, and a ratio of component 2/component 1 of more than 20.

<3> An aspect of the invention is a method of measuring at least one kind of hemoglobin, selected from abnormal hemoglobin, or hemoglobin that is a marker for thalassemia, the method including: performing high-performance liquid chromatography, using: an eluent M that contains a phosphoric acid monohydrogen dialkali metal salt (component 1) and a phosphoric acid dihydrogen monoalkali metal salt (component 2) as components, and that has a content of component 1 of from 0.40% by mass to less than 1.04% by mass with respect to a total mass of the eluent M, a content of component 2 of from more than 0.26% by mass to 0.88% by mass with respect to a total mass of the eluent M, and a ratio of component 2/component 1 of from more than 0.25 to 2.2.

<4> An aspect of the invention is a method of measuring at least one kind of hemoglobin, selected from abnormal hemoglobin, or hemoglobin that is a marker for thalassemia, the method including: performing high-performance liquid chromatography, using: at least two eluents selected from the group consisting of: an eluent L that contains a phosphoric acid monohydrogen dialkali metal salt (component 1) and a phosphoric acid dihydrogen monoalkali metal salt (component 2) as components and that has a content of component 1 of from 0.08% by mass to 0.50% by mass with respect to a total mass of the eluent L, a content of component 2 of from 0.04% by mass to 1.2% by mass with respect to a total mass of the eluent L, and a ratio of component 2/component 1 of from 0.4 to 10; an eluent O that has a content of component 1 of less than 0.07% by mass with respect to a total mass of the eluent O, a content of component 2 of from more than 1.4% by mass to 2.0% by mass with respect to a total mass of the eluent O, and a ratio of component 2/component 1 of more than 20; and an eluent M that has a content of component 1 of from 0.40% by mass to less than 1.04% by mass with respect to a total mass of the eluent M, a content of component 2 of from more than 0.26% by mass to 0.88% by mass with respect to a total mass of the eluent M, and a ratio of component 2/component 1 of from more than 0.25 to 2.2.

<5> An aspect of the invention is the measurement method according to <4>, in which the at least two eluents are used in accordance with any one of the following (I) to (III):

(I) the at least two eluents selected from the group consisting of the eluent L, the eluent O, and the eluent M are used successively without mixing;
(II) at least one mixed liquid prepared by mixing the at least two eluents selected from the group consisting of the eluent L, the eluent O, and the eluent M, is used; or
(III) at least one eluent selected from the group consisting of the eluent L, the eluent O, and the eluent M, and at least one mixed liquid prepared by mixing the at least two eluents selected from the group consisting of the eluent L, the eluent O, and the eluent M, are used successively.

<6> An aspect of the invention is the measurement method according to <4> or <5>, in which at least one mixed liquid prepared by mixing at least one of the eluent O or the eluent M with the eluent L is used.

<7> An aspect of the invention is the measurement method according to any one of <4> to <6>, in which the eluent L, a mixed liquid prepared by mixing the eluent L and the eluent O, and a mixed liquid prepared by mixing the eluent M and the eluent L are used.

<8> An aspect of the invention is the measurement method according to <7>, in which at least one hemoglobin variant of HbBart's, HbH, HbF, HbA1c, HbA0, HbA2, HbE, HbD, HbS, HbC, or HbConstantSpring is measured.

<9> An aspect of the invention is the measurement method according to any one of <1> and <4> to <8>, in which an osmotic pressure of the eluent L is from 35 mOsm to 200 mOsm.

<10> An aspect of the invention is the measurement method according to any one of <2> and <4> to <8>, in which an osmotic pressure of the eluent O is from 170 mOsm to 290 mOsm.

<11> An aspect of the invention is the measurement method according to any one of <3> to <8>, in which in which an osmotic pressure of the eluent M is from 160 mOsm to 210 mOsm.

<12> An aspect of the invention is the measurement method according to any one of <5> to <8>, in which a mixed liquid prepared by mixing the eluent L and the eluent O is used, and a mixing ratio of eluent L:eluent O is from 1:2 to 1:4.

<13> An aspect of the invention is the measurement method according to any one of <5> to <8>, in which a mixed liquid prepared by mixing the eluent M and the eluent L is used, and a mixing ratio of eluent M:eluent L is from 1:2 to 1:4.

<14> An aspect of the invention is the measurement method according to any one of <1> to <13>, further including using an eluent A that has a pH of from 5.30 to 5.40 and an osmotic pressure of from 204 mOsm to 210 mOsm.

<15> An aspect of the invention is the measurement method according to any one of <1> to <14>, further including using an eluent B that has a pH of from 7.85 to 8.25 and an osmotic pressure of from 350 mOsm to 600 mOsm.

<16> An aspect of the invention is the measurement method according to any one of <1> to <13>, further including using a mixed liquid prepared by mixing an eluent B that has a pH of from 7.85 to 8.25 and an osmotic pressure of from 350 mOsm to 600 mOsm and an eluent A that has a pH of from 5.30 to 5.40 and an osmotic pressure of from 204 mOsm to 210 mOsm at a mixing ratio of eluent B:eluent A of from 1:2 to 1:4.

<17> An aspect of the invention is the measurement method according to <16>, in which at least one hemoglobin variant of HbBart's, HbH, HbF, HbA1c, HbA0, HbA2, HbE, HbD, HbS, HbC, or HbConstantSpring is measured.

<18> An aspect of the invention is a measurement apparatus for measuring a hemoglobin variant by high-performance liquid chromatography, the measurement apparatus including:

a supply pump to supply, singly or in combination, one or more eluents selected from the group consisting of an eluent L that contains a phosphoric acid monohydrogen dialkali metal salt (component 1) and a phosphoric acid dihydrogen monoalkali metal salt (component 2) as components and that has a content of component 1 of from 0.08% by mass to 0.50% by mass with respect to a total mass of the eluent L, a content of component 2 of from 0.04% by mass to 1.2% by mass with respect to a total mass of the eluent L, and a ratio of component 2/component 1 of from 0.4 to 10, an eluent O that has a content of component 1 of less than 0.07% by mass with respect to a total mass of the eluent O, a content of component 2 of from more than 1.4% by mass to 2.0% by mass with respect to a total mass of the eluent O, and a ratio of component 2/component 1 of more than 20, and an eluent M that has a content of component 1 of from 0.40% by mass to less than 1.04% by mass with respect to a total mass of the eluent M, a content of component 2 of from more than 0.26% by mass to 0.88% by mass with respect to a total mass of the eluent M, and a ratio of component 2/component 1 of from more than 0.25 to 2.2;

a separation column which is connected to the supply pump and to which the eluent is supplied; and

a plurality of eluent tanks storing each of eluent L, eluent O and eluent M.

<19> An aspect of the invention is the measurement apparatus according to <18>, in which the supply pump supplies eluents in accordance with any one of the following (I) to (III):

(I) at least one eluent selected from the group consisting of the eluent L, the eluent O, and the eluent M is supplied, or at least two eluents selected from the group consisting of the eluent L, the eluent O, and the eluent M are supplied successively without mixing;

(II) at least one mixed liquid prepared by mixing the at least two eluents selected from the group consisting of the eluent L, the eluent O, and the eluent M, is supplied; and

(III) at least one eluent selected from the group consisting of the eluent L, the eluent O, and the eluent M, and at least one mixed liquid prepared by mixing the at least two eluents selected from the group consisting of the eluent L, the eluent O, and the eluent M, are supplied successively.

<20> An aspect of the invention is the measurement apparatus according to <18> or <19>, in which, as the eluent, at least one of an eluent A that has a pH of from 5.30 to 5.40 and an osmotic pressure of from 204 mOsm to 210 mOsm or an eluent B that has a pH of from 7.85 to 8.25 and an osmotic pressure of from 350 mOsm to 600 mOsm, is further supplied, or a mixed liquid prepared by mixing the eluent A and the eluent B is further supplied.

<21> An aspect of the invention is the measurement apparatus according to any one of <18> to <20>, in which the apparatus has a single supply pump.

<22> An aspect of the invention is an eluent (eluent L) suitable for measuring at least one kind of hemoglobin selected from abnormal hemoglobin or hemoglobin that is a marker for thalassemia by high-performance liquid chromatography, the eluent including:

a phosphoric acid monohydrogen dialkali metal salt (component 1); and

a phosphoric acid dihydrogen monoalkali metal salt (component 2), in which

a content of component 1 is from 0.08% by mass to 0.50% by mass with respect to a total mass of the eluent L, a content of component 2 is from 0.04% by mass to 1.2% by mass with respect to a total mass of the eluent L, and a ratio of component 2/component 1 is from 0.4 to 10.

<23> An aspect of the invention is an eluent (eluent O) suitable for measuring at least one kind of hemoglobin selected from abnormal hemoglobin or hemoglobin that is a marker for thalassemia by high-performance liquid chromatography, the eluent including:

a phosphoric acid monohydrogen dialkali metal salt (component 1); and
a phosphoric acid dihydrogen monoalkali metal salt (component 2), in which
a content of component 1 is less than 0.07% by mass with respect to a total mass of the eluent O,
a content of component 2 is from more than 1.4% by mass to 2.0% by mass with respect to a total mass of the eluent O, and
a ratio of component 2/component 1 is more than 20.

<24> An aspect of the invention is an eluent (eluent M) suitable for measuring at least one kind of hemoglobin selected from abnormal hemoglobin or hemoglobin that is a marker for thalassemia by high-performance liquid chromatography, the eluent including:

a phosphoric acid monohydrogen dialkali metal salt (component 1); and
a phosphoric acid dihydrogen monoalkali metal salt (component 2), in which
a content of component 1 is from 0.40% by mass to less than 1.04% by mass with respect to a total mass of the eluent M,
a content of component 2 is from more than 0.26% by mass to 0.88% by mass with respect to a total mass of the eluent M, and
a ratio of component 2/component 1 is from more than 0.25 to 2.2.

<25> An aspect of the invention is the eluent according to any one of <22> to <24>, which is suitable for measuring at least one hemoglobin variant of HbBart's, HbH, HbF, HbA1c, HbA0, HbA2, HbE, HbD, HbS, HbC, or HbConstantSpring.

<26> An aspect of the invention is the eluent according to <22>, in which an osmotic pressure of the eluent L is from 35 mOsm to 200 mOsm.

<27> An aspect of the invention is the eluent according to <23>, in which an osmotic pressure of the eluent O is from 170 mOsm to 290 mOsm.

<28> An aspect of the invention is the eluent according to <24>, in which an osmotic pressure of the eluent M is from 160 mOsm to 210 mOsm.

<29> An aspect of the invention is the eluent according to any one of <22> to <28>, which is suitable for measuring at least one hemoglobin variant of HbBart's, HbH, HbF, HbA1c, HbA0, HbA2, HbE, HbD, HbS, HbC, or HbConstantSpring.

<30> An aspect of the invention is a combination of eluents suitable for measuring at least one kind of hemoglobin selected from abnormal hemoglobin or hemoglobin that is a marker for thalassemia by high-performance liquid chromatography, the combination of eluents including,

at least two eluents selected from the group consisting of an eluent L that contains a phosphoric acid monohydrogen dialkali metal salt (component 1) and a phosphoric acid dihydrogen monoalkali metal salt (component 2) as components and that has a content of component 1 of from 0.08% by mass to 0.50% by mass with respect to a total mass of the eluent L, a content of component 2 of from 0.04% by mass to 1.2% by mass with respect to a total mass of the eluent L, and a ratio of component 2/component 1 of from 0.4 to 10; an eluent O that has a content of component 1 of less than 0.07% by mass with respect to a total mass of the eluent O, a content of component 2 of from more than 1.4% by mass to 2.0% by mass with respect to a total mass of the eluent O, and a ratio of component 2/component 1 of more than 20; and an eluent M that has a content of component 1 of from 0.40% by mass to less than 1.04% by mass with respect to a total mass of the eluent M, a content of component 2 of from more than 0.26% by mass to 0.88% by mass with respect to a total mass of the eluent M, and a ratio of component 2/component 1 of from more than 0.25 to 2.2.

<31> An aspect of the invention is the combination of eluents according to <30>, in which at least two eluents selected from the group consisting of the eluent L, the eluent O, and the eluent M are in a state of not being mixed.

<32> An aspect of the invention is the combination of eluents according to <30>, the combination of eluents including an eluent that satisfies the following (a) and a mixed liquid that satisfies the following (b), in which

the eluent that satisfies the following (a) and the mixed liquid that satisfies the following (b) correspond to at least one of a state of not being mixed or a state of being mixed:

(a) at least one eluent selected from the group consisting of the eluent L, the eluent O, and the eluent M; and
(b) at least one mixed liquid prepared by mixing two or more kinds of eluents selected from the group consisting of the eluent L, the eluent O, and the eluent M.

<33> An aspect of the invention is the combination of eluents according to <30>, the combination of eluents including:

the eluent L;

a mixed liquid prepared by mixing the eluent L and the eluent O; and

a mixed liquid prepared by mixing the eluent M and the eluent L.

<34> An aspect of the invention is the combination of eluents according to any one of <30> to <33>, which is suitable for measuring at least one hemoglobin variant of HbBart's, HbH, HbF, HbA1c, HbA0, HbA2, HbE, HbD, HbS, HbC, or HbConstantSpring.

<35> An aspect of the invention is the combination of eluents according to any one of <30> to <34>, the combination of eluents including the eluent L, in which an osmotic pressure of the eluent L is from 35 mOsm to 200 mOsm.

<36> An aspect of the invention is the combination of eluents according to any one of <30> to <35>, the combination of eluents including the eluent O, in which an osmotic pressure of the eluent O is from 170 mOsm to 290 mOsm.

<37> An aspect of the invention is the combination of eluents according to any one of <30> to <36>, the combination of eluents including the eluent M, in which an osmotic pressure of the eluent M is from 160 mOsm to 210 mOsm.

<38> An aspect of the invention is the combination of eluents according to any one of <30> to <37>, the combination of eluents including a mixed liquid prepared by mixing the eluent L and the eluent O, in which a mixing ratio of the mixed liquid is eluent L: eluent O of from 1:2 to 1:4.

<39> An aspect of the invention is the combination of eluents according to any one of <30> to <38>, the combination of eluents including a mixed liquid prepared by mixing the eluent M and the eluent L, in which a mixing ratio of the mixed liquid is eluent M : eluent L of from 1:2 to 1:4.

<40> An aspect of the invention is the combination of eluents according to any one of <30> to <39>, which is suitable for measuring at least one hemoglobin variant of HbBart's, HbH, HbF, HbA1c, HbA0, HbA2, HbE, HbD, HbS, HbC, or HbConstantSpring.

<41>An aspect of the invention is at least one mixed liquid prepared by mixing two or more kinds of eluents selected from the group consisting of the eluent L, the eluent O, and the eluent M in the combination according to <30>.

<42> An aspect of the invention is the mixed liquid according to <41>, in which the mixed liquid is at least one mixed liquid prepared by mixing at least one of the eluent O or the eluent M with the eluent L.

<43> An aspect of the invention is the mixed liquid according to <41> or <42>, which is suitable for measuring at least one hemoglobin variant of HbBart's, HbH, HbF, HbA1c, HbA0, HbA2, HbE, HbD, HbS, HbC, or HbConstant-Spring.

<44> An aspect of the invention is the mixed liquid according to any one of <41> to <43>, the mixed liquid including the eluent L, in which an osmotic pressure of the eluent L is from 35 mOsm to 200 mOsm.

<45> An aspect of the invention is the mixed liquid according to any one of <41> to <44>, the mixed liquid including the eluent O, in which an osmotic pressure of the eluent O is from 170 mOsm to 290 mOsm.

<46> An aspect of the invention is the mixed liquid according to any one of <41> to <45>, the mixed liquid including the eluent M, in which an osmotic pressure of the eluent M is from 160 mOsm to 210 mOsm.

<47> An aspect of the invention is the mixed liquid according to any one of <41> to <46>, in which

the mixed liquid is prepared by mixing the eluent L and the eluent O, and

a mixing ratio of the eluents is eluent L : eluent O of from 1:2 to 1:4.

<48> An aspect of the invention is the mixed liquid according to any one of <41> to <47>, in which

a mixed liquid prepared by mixing the eluent M and the eluent L, and

a mixing ratio of the eluents is eluent M : eluent L of from 1:2 to 1:4.

<49> An aspect of the invention is the mixed liquid according to any one of <41> to <48>, which is suitable for measuring at least one hemoglobin variant of HbBart's, HbH, HbF, HbA1c, HbA0, HbA2, HbE, HbD, HbS, HbC, or HbConstantSpring.

<50> An aspect of the invention is the measurement method according to any one of <1> to <17>, in which the eluent is supplied to a separation column.

<51> An aspect of the invention is the measurement method according to <50>, in which a flow rate when supplying the eluent to the separation column is 0.001 ml/min or more.

<52> An aspect of the invention is the measurement method according to <50> or <51>, in which a flow rate when supplying the eluent to the separation column is 5.0 ml/min or less.

<53> An aspect of the invention is the measurement method according to any one of <5> to <8>, <12>, <13>, <16>, and <17>, in which the mixed liquid is supplied to a separation column.

<54> An aspect of the invention is the measurement method according to <53>, in which a flow rate when supplying the mixed liquid to the separation column is 0.001 ml/min or more.

<55> An aspect of the invention is the measurement method according to <53> or <54>, in which a flow rate when supplying the mixed liquid to the separation column is 5.0 ml/min or less.

<56> An aspect of the invention is the measurement apparatus according to any one of <18> to <21>, in which a flow rate when supplying the eluent to the separation column is 0.001 ml/min or more.

<57> An aspect of the invention is the measurement apparatus according to any one of <18> to <21>, and <56>, in which a flow rate when supplying the eluent to the separation column is 5.0 ml/min or less.

<58> An aspect of the invention is the measurement apparatus according to <19> or <20>, in which a flow rate when supplying the mixed liquid to the separation column is 0.001 ml/min or more.

<59> An aspect of the invention is the measurement apparatus according to any one of <19>, <20>, and <58>, in which a flow rate when supplying the mixed liquid to the separation column is 5.0 ml/min or less.

<60> An aspect of the invention is the measurement apparatus according to any one of <18> to <21> and <56> to <59>, in which the separation column is a cation exchange column.

<61> An aspect of the invention is the measurement apparatus according to <60>, in which a cation exchange group of the cation exchange column is at least any one of a sulfo group, a carboxyl group, or a phosphoric acid group.

<62> An aspect of the invention is the measurement apparatus according to <60>, in which a cation exchange group of the cation exchange column is a sulfo group.

<63> An aspect of the invention is the measurement apparatus according to any one of <18> to <21> and <56> to <62>, in which a column packing material of the separation column is a polymer gel or an inorganic gel.

<64> An aspect of the invention is the measurement apparatus according to <63>, in which the column packing material is a polymer gel, and the polymer gel is a methacrylic acid-methacrylic acid ester copolymer.

<65> An aspect of the invention is the measurement apparatus according to any one of <18> to <21> and <56> to <64>, in which a length of the separation column is 1 mm or more.

<66> An aspect of the invention is the measurement apparatus according to any one of <18> to <21> and <56> to <65>, in which a length of the separation column is 300 mm or less.

<67> An aspect of the invention is the measurement apparatus according to any one of <18> to <21> and <56> to <66>, in which an inner diameter of the separation column is 0.1 mm or more.

<68> An aspect of the invention is the measurement apparatus according to any one of <18> to <21> and <56> to <67>, in which an inner diameter of the separation column is 50 mm or less.

<69> An aspect of the invention is the measurement apparatus according to any one of <18> to <21> and <56> to <68>, in which an average particle size of a column packing material in the separation column is 0.1 $\mu$m or more.

<70> An aspect of the invention is the measurement apparatus according to any one of <18> to <21> and <56> to <69>, in which an average particle size of a column packing material in the separation column is 50 $\mu$m or less.

According to an aspect of the invention, it is possible to provide a measurement method, a measurement apparatus and an eluent that can measure at least one kind of hemoglobin selected from abnormal hemoglobin or hemoglobin that is a marker for thalassemia.

BRIEF DESCRIPTION OF THE DRAWINGS

Fig. 1 illustrates a measurement apparatus for measuring hemoglobin variants according to the present embodiment.

Fig. 2 illustrates the measurement results in the case of using a healthy individual specimen as a measurement sample of Example 1.

Fig. 3 illustrates the measurement results in the case of using a specimen containing HbBart's, HbF, HbA0, HbE, HbD, HbS, HbC and HbCS as the measurement sample of Example 1.

Fig. 4 illustrates the measurement results in the case of using a specimen containing HbBart's and HbH of Example 1.

Fig. 5 illustrates the measurement results in the case of using a specimen containing HbBart's, HbF, HbA1c, HbA0, HbE, HbD, HbS, HbC and HbCS as the measurement sample of Example 2.

Fig. 6 illustrates the measurement results in the case of using a specimen containing HbE and a healthy individual specimen as the measurement sample of Example 3.

Fig. 7 illustrates the measurement results in the case of using a specimen containing HbE and a healthy individual specimen as the measurement sample and comparative eluent L instead of eluent L of Example 4.

Fig. 8 illustrates the measurement results in the case of using a specimen containing HbBart's as the measurement sample of Example 5.

Fig. 9 illustrates the measurement results in the case of using a specimen containing HbBart's as the measurement sample and comparative eluent O instead of eluent O of Example 6.

Fig. 10 illustrates the measurement results in the case of using a specimen containing HbS and HbC and a specimen containing HbCS as the measurement samples of Example 7.

Fig. 11 illustrates the measurement results in the case of using a specimen containing HbS and HbC and a specimen containing HbCS as the measurement samples and comparative eluent M instead of eluent M of Example 8.

Fig. 12 illustrates the relationship between the osmotic pressure and the pH in eluent L.

Fig. 13 illustrates the relationship between the osmotic pressure and the ratio of component 2/component 1 in eluent L.

Fig. 14 illustrates the relationship between the osmotic pressure and the pH in eluent M.

Fig. 15 illustrates the relationship between the osmotic pressure and the ratio of component 2/component 1 in eluent M.

Fig. 16 illustrates the relationship between the osmotic pressure and the pH in eluent O.

Fig. 17 illustrates the relationship between the osmotic pressure and the ratio of component 2/component 1 in eluent O.

Fig. 18 illustrates the relationship between the osmotic pressure and the pH in eluent B.

DETAILED DESCRIPTION OF THE INVENTION

Hereinafter, embodiments according to the measurement method of an aspect of the invention will be described in detail.

In an embodiment according to the measurement method of an aspect of the invention, it is possible to measure at least one kind of hemoglobin of glycosylated hemoglobin (HbA1c), hemoglobin A0 (HbA0), abnormal hemoglobin (abnormal Hb), or hemoglobin that is a marker for thalassemia by high-performance liquid chromatography (HPLC) using an eluent singly or a plurality of eluents as a mixture. Incidentally, examples of the high-performance liquid chromatography method include a cation exchange chromatography method, an anion exchange chromatography method, a partition chromatography method, a reversed-phase partition chromatography method, an affinity chromatography method, and a gel filtration chromatography method.

HbA1c is a kind of glycated protein in which hemoglobin is bound to glucose and is an indicator for diabetes diagnosis. In addition, HbA0 accounts for most of the hemoglobin in the blood sample and accounts for about 90% of the total hemoglobin in a healthy individual. In addition, there is HbF or HbA2 as hemoglobin that is contained in a healthy individual.

Examples of the abnormal Hb include HbE, HbS, HbD, and HbC. The measurable abnormal Hb varies depending on the pH and osmotic pressure of the eluent or a mixed liquid prepared by mixing a plurality of eluents, and the concentration of each component.

In addition, in an embodiment according to the measurement method of an aspect of the invention, it is possible to measure hemoglobin that is a marker for thalassemia by high-performance liquid chromatography (HPLC) using an eluent singly or a plurality of eluents as a mixture. Examples of the hemoglobin that is a marker for thalassemia include HbBart's, HbH, and HbConstantSpring (hereinafter, also referred to as "HbCS").

Incidentally, HbA1c, HbA0, HbF, HbA2, abnormal hemoglobin, and hemoglobin that is a marker for thalassemia are generally referred to as the "hemoglobin variants" hereinafter.

In the measurement method according to an aspect of the invention, various kinds of hemoglobin variants as described above are the target to be measured. Moreover, it is possible to separate and measure the hemoglobin variants that are not the target to be separated and measured by the techniques of JP-A No. H05-281222 and JP-A No. 2009-236768 described above.

The sample that is used in an aspect of the invention refers to those which are prepared from a sample raw material. The sample raw material is a biological sample containing a hemoglobin variant. Examples of the biological sample include blood, a blood-derived product containing a red blood cell component, saliva, and cerebrospinal fluid. As the blood, blood collected from a living body is mentioned, and the blood is preferably blood of an animal, more preferably blood of Mammalia, and still more preferably human blood. As the blood-derived product containing a red blood cell component, those which are separated or prepared from blood and contain a red blood cell component are mentioned and examples thereof include a blood cell fraction from which blood plasma is removed, a blood cell concentrate, a freeze-dried matter of blood or blood cell, a hemolyzed sample prepared by subjecting whole blood to the hemolysis treatment, blood prepared by centrifugal separation, and blood prepared by spontaneous sedimentation.

The sample that is used in an aspect of the invention may be those which are prepared by diluting a sample raw material. In addition, the concentration (content) of the sample raw material in the sample is preferably from 1% by mass to 30% by mass from the viewpoint of the suppression of hemoglobin denaturation.

(Eluent L)

The eluent L used in the measurement method of an aspect of the invention is an eluent that contains a phosphoric acid monohydrogen dialkali metal salt (component 1) and a phosphoric acid dihydrogen monoalkali metal salt (component 2) as components and that has a content of component 1 of from 0.08% by mass to 0.50% by mass with respect to a total mass of the eluent L, a content of component 2 of from 0.04% by mass to 1.2% by mass with respect to a total mass of the eluent L, and a ratio of component 2/component 1 of from 0.4 to 10.

It is possible to measure abnormal hemoglobin, hemoglobin that is a marker for thalassemia, and the like by using the eluent L in the measurement of hemoglobin variants singly or in combination with other eluents. In addition, for example, it is possible to simultaneously measure at least two kinds of hemoglobin of normal hemoglobin, abnormal hemoglobin, and hemoglobin that is a marker for thalassemia, for example, it is possible to collectively measure at least one of the hemoglobin variants such as HbBart's, HbH, HbF, HbA1c, HbA0, HbA2, HbE, HbD, HbS, HbC, and HbConstantSpring, and in particular, it is possible to collectively measure at least one of the hemoglobin variants such as HbBart's, HbH, HbF, HbA0, HbE, HbA2, HbD, and HbS. In particular, it is possible to collectively measure hemoglobin variants such as HbA0, HbE, HbA2, and HbD by using the eluent L singly. Incidentally, Hb that is eluted before Hb of the target to be measured can be eluted by another eluent, if necessary.

Incidentally, in the measurement method of measuring hemoglobin variants according to an aspect of the invention, a sample is injected into a separation column, an eluent is then supplied to the separation column, and the hemoglobin contained in the sample is separated and measured according to the eluent supplied.

In addition, in a case in which the eluent L is supplied to separate, for example, HbA0, HbE, and HbD, it is possible to separate each of these hemoglobin variants with high accuracy according to the measurement time, for example, as the elution times of these hemoglobin variants from the separation column do not overlap with one another. Furthermore, in a case in which the eluent L is supplied to separate, for example, HbA0, HbA2, and HbD as well, it is possible to separate each of these hemoglobin variants with high accuracy according to the measurement time, for example, as the elution times of these hemoglobin variants from the separation column do not overlap with one another.

The ratio of the component 1 to the component 2 of the eluent L is not limited as long as the above numerical range is satisfied, but the content of component 1 is preferably from 0.15% by mass to 0.40% by mass, more preferably from 0.20% by mass to 0.29% by mass, and still more preferably from 0.20% by mass to 0.27% by mass. The content of component 2 is preferably from 0.35% by mass to 1.05% by mass, and more preferably from 0.60% by mass to 0.70% by mass. The ratio of component 2/component 1 is preferably from 1.4 to 7.0, and more preferably from 2.2 to 3.5.

The osmotic pressure of the eluent L is preferably from 35 mOsm to 200 mOsm, more preferably from 80 mOsm to 185 mOsm, and still more preferably from 125 mOsm to 140 mOsm. The osmotic pressure of the eluent L or the respective eluents presented below can be adjusted by changing the concentrations of the phosphoric acid monohydrogen dialkali metal salt (component 1) and the phosphoric acid dihydrogen monoalkali metal salt (component 2).

The pH of the eluent L is preferably from 5.5 to 7.4, more preferably from 5.8 to 6.7, and still more preferably from 6.2 to 6.4.

It is preferable to prepare the eluent L by adding other additives in addition to the component 1 and component 2 described above. As the other additives, for example, a buffering agent, an inorganic salt, a pH adjusting agent, a water-soluble organic solvent, a preservative, a hemoglobin stabilizer and a chaotropic ion may be added. In addition, the eluent L may be diluted with distilled water and the like.

Examples of the buffering agent to be added to the eluent L include an organic substance such as a carboxylic acid, a dicarboxylic acid, a carboxylic acid derivative, hydroxycarboxylic acid, aniline or an aniline derivative, an amino acid, an amine, an imidazole, or an alcohol in addition to an inorganic substance such as phosphoric acid, boric acid, or carbonic acid. In addition, the buffering agent may be an organic substance such as ethylenediaminetetraacetic acid, pyrophosphoric acid, pyridine, cacodylic acid, glycerol phosphate, 2,4,6-collidine, N-ethylmorpholine, morpholine, 4-aminopyridine, ammonia, ephedrine, hydroxyproline, piperidine, tris(hydroxymethyl)aminomethane, or glycylglycine. The content of the buffering agent in the eluent L may be in any range as long as the buffering effect is exhibited, and it is preferably from 1 mM to 1000 mM and more preferably from 10 mM to 500 mM. In addition, a kind of the buffering agents may be used or kinds of the agents may be used concurrently, and for example, an organic substance and an inorganic substance may be used concurrently.

Examples of the inorganic salt to be added to the eluent L include sodium chloride, potassium chloride, sodium sulfate, potassium sulfate, and sodium phosphate. It is possible to optimize the peak elution of the hemoglobin variants by adding an inorganic salt to the eluent L. The concentration of these inorganic salts is not particularly limited, and it is preferably from 1 mM to 1500 mM.

Examples of the pH adjusting agent to be added to the eluent L include an acid and a base which are known. Examples of the acid include hydrochloric acid, phosphoric acid, nitric acid, and sulfuric acid, and examples of the base include sodium hydroxide, potassium hydroxide, lithium hydroxide, magnesium hydroxide, barium hydroxide, and calcium hydroxide. The concentration of these acids and bases is not particularly limited, and it is preferably from 0.001 mM to 500 mM.

Examples of the water-soluble organic solvent to be added to the eluent L include methanol, ethanol, 2-phenoxyethanol, acetonitrile, and acetone. As the concentration of these water-soluble organic solvents, it is preferable to use these water-soluble organic solvents to the extent to which a salt and the like do not precipitate, and the preferred upper limit thereof is 80% (v/v).

Examples of the preservative to be added to the eluent L include sodium azide, thymol, and sodium propionate. The concentration of these preservatives is not particularly limited, and it is preferably from 0.1 mM to 100 mM.

In addition, examples of the hemoglobin stabilizer to be added to the eluent L include a known stabilizer, for example, a chelating agent such as ethylenediaminetetraacetic acid (EDTA), or a reductant or an antioxidant such as glutathione or sodium azide. The concentration of these hemoglobin stabilizers is not particularly limited, and it is preferably from 0.1 mM to 100 mM.

In addition, a chaotropic ion may be added to the eluent L. The chaotropic ion is an ion which is generated by the dissociation when a compound is dissolved in an aqueous solution, breaks the structure of water, and suppresses a decrease in entropy of water occurring when a hydrophobic substance comes in contact with water.

As the chaotropic ion to be added to the eluent L, there is a chaotropic ion of an anion and a cation. Examples of the chaotropic ion of an anion include tribromoacetate ion, trichloroacetate ion, thiocyanate ion, iodide ion, perchlorate ion, dichloroacetate ion, nitrate ion, bromide ion, chloride ion, and acetate ion, and examples of the chaotropic ion of a cation include barium ion, calcium ion, lithium ion, a cesium ion, potassium ion, magnesium ion, and guanidinium ion. The content of the chaotropic ion in the eluent L is preferably from 0.1 mM to 3000 mM, more preferably from 1 mM to 1000 mM, and still more preferably from 10 mM to 500 mM. The separation effect increases in the measurement of the hemoglobin variants as the content is 0.1 mM or more, and it is possible to efficiently improve the separation effect of the hemoglobin variants as the content is 3000 mM or less.

(Eluent O)

The eluent O used in the measurement method of an aspect of the invention is an eluent that contains a phosphoric acid monohydrogen dialkali metal salt (component 1) and a phosphoric acid dihydrogen monoalkali metal salt (component 2) as components and that has a content of component 1 of less than 0.07% by mass with respect to a total mass of the eluent O, a content of component 2 of from more than 1.4% by mass to 2.0% by mass with respect to a total mass of the eluent O, and a ratio of component 2/component 1 of more than 20.

It is possible to measure at least one of the hemoglobin variants such as HbBart's, HbH, HbF, HbA1c, HbA0, HbA2, HbE, HbD, HbS, HbC, and HbConstantSpring by using the eluent O in the measurement of hemoglobin variants singly or in combination with other eluents, and in particular, it is possible to measure hemoglobin variants such as HbBart's, HbH, HbF, and HbA1c. It is possible to measure hemoglobin that is a marker for thalassemia such as HbBart's by using the eluent O singly. Incidentally, Hb that is eluted before Hb of the target to be measured can be eluted by another eluent, if necessary.

The ratio of the component 1 to the component 2 of the eluent O is not limited as long as the above numerical range is satisfied, but the content of component 1 is preferably 0.05% by mass or less, more preferably 0.04% by mass or less, and still more preferably 0.03% by mass or less. The lower limit of the content of component 1 is not particularly limited, and it is preferably 0.001% by mass or more, more preferably 0.002% by mass or more, and still more preferably 0.01% by mass or more. Furthermore, the content of component 2 is preferably from 1.42% by mass to 1.70% by mass and more preferably from 1.44% by mass to 1.60% by mass. The ratio of component 2/component 1 is preferably 25 or more, more preferably 40 or more, and still more preferably 48 or more. The upper limit of the ratio of component 2/component 1 is preferably 1000 or less and more preferably 300 or less.

The osmotic pressure of the eluent O is preferably from 170 mOsm to 290 mOsm, more preferably from 190 mOsm to 220 mOsm, and still more preferably from 200 mOsm to 210 mOsm.

The pH of the eluent O is preferably 5.4 or less, more preferably from 4.8 to 5.4, and still more preferably from 5.0 to 5.3.

It is preferable to prepare the eluent O which satisfies the above numerical range by adding other additives in addition to the phosphoric acid-based buffer solution containing the component 1 and the component 2. The other additives are the same as those in the case of preparing the eluent L described above.

(Eluent M)

The eluent M used in the measurement method of measuring hemoglobin variants according to an aspect of the invention is an eluent that contains a phosphoric acid monohydrogen dialkali metal salt (component 1) and a phosphoric acid dihydrogen monoalkali metal salt (component 2) as components and that has a content of component 1 of from 0.40% by mass to less than 1.04% by mass with respect to a total mass of the eluent M, a content of component 2 of from more than 0.26% by mass to 0.88% by mass with respect to a total mass of the eluent M, and a ratio of component 2/component 1 of from more than 0.25 to 2.2.

It is possible to measure at least one kind of hemoglobin of normal hemoglobin, abnormal hemoglobin, or hemoglobin that is a marker for thalassemia such as HbBart's, HbH, HbF, HbA1c, HbA0, HbE, HbA2, HbD, HbS, HbC, or HbCS by using the eluent M in the measurement of hemoglobin variants singly or in combination with other eluents. In particular, it is possible to measure abnormal hemoglobin such as HbC, and hemoglobin that is a marker for thalassemia such as HbCS by using the eluent M singly. Incidentally, Hb that is eluted before Hb of the target to be measured can be eluted by another eluent, if necessary. Incidentally, in more detail, it is possible to separate each of the measurable hemoglobin variants such as HbC and HbCS with high accuracy according to the measurement time, for example, as the elution times of these hemoglobin variants from the separation column do not overlap with one another.

The ratio of the component 1 to the component 2 of the eluent M is not limited as long as the above numerical range is satisfied, but the content of component 1 is preferably from 0.5% by mass to 0.90% by mass and more preferably from 0.6% by mass to 0.80% by mass, and the content of component 2 is preferably from 0.45% by mass to 0.75% by mass and more preferably from 0.55% by mass to 0.65% by mass. The ratio of component 2/component 1 is preferably from 0.5 to 1.7 and more preferably from 0.7 to 1.4.

The osmotic pressure of the eluent M is preferably from 160 mOsm to 210 mOsm, more preferably from 170 mOsm to 200 mOsm, and still more preferably from 175 mOsm to 190 mOsm.

The pH of the eluent M is preferably 7.3 or less, more preferably from 6.4 to 7.0, and still more preferably from 6.5 to 6.9.

It is preferable to prepare the eluent M which satisfies the above numerical range by adding other additives in addition to the phosphoric acid-based buffer solution containing the component 1 and the component 2. The other additives are the same as those in the case of preparing the eluent L described above.

(Eluent A)

In the measurement method according to an aspect of the invention, an eluent (eluent A) that has a pH of from 5.30 to 5.40 and an osmotic pressure of from 204 mOsm to 210 mOsm may further be used. The eluent A can be obtained by changing the concentrations of the component 1 and the component 2 so as to adjust the pH and the osmotic pressure.

It is possible to collectively measure at least one of HbBart's, HbH, HbF, HbA1c, HbA0, HbA2, HbE, HbD, HbS, HbC, or HbConstantSpring by using the eluent A in the measurement of hemoglobin variants singly or in combination with other eluents, and in particular, it is possible to collectively measure HbBart's, HbH, HbF, HbA1c, HbA0, HbA2, HbE, HbD, HbS, HbC, and HbCS. In particular, it is possible to collectively measure HbF and HbA1c by using the eluent A singly. The eluent A may be used as a mixed liquid prepared by mixing with any of the eluent L, the eluent O, or the eluent M described above, and also the eluent A in combination with the eluent L, the eluent O, and the eluent M may be supplied to the separation column successively. The collective separation of HbA1c and abnormal Hb, or the collective separation of HbA1c and hemoglobin that is a marker for thalassemia is possible as the eluent A is supplied in combination with the eluent L, the eluent O, and the eluent M. Among them, it is possible to collectively measure HbA1c and abnormal Hb by supplying the eluent L and eluent A in combination, and thus it is useful. Incidentally, Hb that is eluted before Hb of the target to be measured can be eluted by another eluent, if necessary.

(Eluent B)

In the measurement method according to an aspect of the invention, an eluent (eluent B) that has a pH of from 7.85 to 8.25 and an osmotic pressure of from 350 mOsm to 600 mOsm may further be used. The eluent B can be obtained by changing the concentrations of the component 1 and the component 2 so as to adjust the pH and the osmotic pressure. In addition, the osmotic pressure of the eluent B is preferably from 370 mOsm to 570 mOsm and more preferably from 400 mOsm to 480 mOsm.

It is possible to collectively measure at least one of HbBart's, HbH, HbF, HbA1c, HbA0, HbA2, HbE, HbD, HbS, HbC, or HbConstantSpring by using a mixed liquid prepared by mixing the eluent B and the eluent A in the measurement of hemoglobin variants, and in particular, it is possible to collectively measure HbS, HbC and the HbCS. In addition, the eluent B may be used as a mixed liquid prepared by mixing with any of the eluent L, the eluent O, or the eluent M described above. Moreover, the eluent B may be supplied to the separation column after the eluent L, the eluent O, the eluent M, or a mixed liquid thereof is supplied to the separation column successively so as to be used for flushing the sample in the separation column. Incidentally, Hb that is eluted before Hb of the target to be measured can be eluted by another eluent, if necessary.

The eluent L, the eluent O, the eluent M, the eluent A and the eluent B described above may successively be supplied singly, a mixed liquid prepared by mixing any of the eluent L, the eluent O, the eluent M, the eluent A, or the eluent B described above may successively be supplied, or the eluents and the mixed liquids in combination may successively be supplied. It is possible to collectively measure various kinds of hemoglobin variants by supplying the eluents and the mixed liquids in combination.

Among them, in the case of collectively measuring various kinds of hemoglobin variants, it is preferable to supply at least two eluents selected from the group consisting of an eluent (eluent L) that contains a phosphoric acid monohydrogen dialkali metal salt (component 1) and a phosphoric acid dihydrogen monoalkali metal salt (component 2) as components and that has a content of component 1 of from 0.08% by mass to 0.50% by mass, a content of component 2 of from 0.04% by mass to 1.2% by mass, and a ratio of component 2/component 1 of from 0.4 to 10, an eluent (eluent O) that has a content of component 1 of less than 0.07% by mass, a content of component 2 of from more than 1.4% by mass to 2.0% by mass, and a ratio of component 2/component 1 of more than 20, and an eluent (eluent M) that has a content of component 1 of from 0.40% by mass to less than 1.04% by mass, a content of component 2 of from more than 0.26% by mass to 0.88% by mass, and a ratio of component 2/component 1 of from more than 0.25 to 2.2 in combination in high-performance liquid chromatography. Incidentally, Hb that is eluted before Hb of the target to be measured can be eluted by another eluent, if necessary.

As the method of measuring hemoglobin variants, it is preferable to supply the eluents in accordance with any one of the following (I) to (III): (I) at least one eluent selected from the group consisting of the eluent L, the eluent O, and the eluent M is supplied, or at least two eluents selected from the group consisting of the eluent L, the eluent O, and the eluent M are supplied successively without mixing, (II) at least one mixed liquid prepared by mixing two or more kinds of eluents selected from the group consisting of the eluent L, the eluent O, and the eluent M is supplied, and (III) at least one eluent selected from the group consisting of the eluent L, the eluent O, and the eluent M, and at least one mixed liquid prepared by mixing two or more kinds of eluents selected from the group consisting of the eluent L, the eluent O, and the eluent M are supplied successively. This makes it possible to collectively measure various kinds of hemoglobin variants. Incidentally, for (III) described above, the order of supplying the eluent and the mixed liquid is not particularly limited, and they may be supplied in an arbitrary order. Hence, for example, the eluent may be supplied first, the mixed liquid may be supplied first, or the eluent and the mixed liquid may be randomly supplied. Incidentally, Hb that is eluted before Hb of the target to be measured can be eluted by another eluent, if necessary.

In the case of supplying plural kinds of eluents and mixed liquids, either of a linear gradient method or a step gradient method may be adopted.

The flow rate of the respective eluents or the respective mixed liquids when being supplied to the separation column is, for example, preferably 0.001 ml/min or more, more preferably 0.01 ml/min or more, still more preferably 0.05 ml/min or more, still more preferably 0.1 ml/min or more, still more preferably 0.2 ml/min or more, still more preferably 0.3 ml/min or more, still more preferably 1.0 ml/min or more, and still more preferably 1.5 ml/min or more. In addition, the flow rate of the respective eluents or the respective mixed liquids when being supplied to the separation column is, for example, preferably 5.0 ml/min or less, more preferably 4.0 ml/min or less, still more preferably 3.0 ml/min or less, and particularly preferably 2.5 ml/min or less.

The time to supply the respective eluents or the respective mixed liquids to the separation column is not particularly limited, and the time may be determined depending on the kind of the hemoglobin variants of the target to be measured. For the eluent L, the time is preferably from 21 seconds to 105 seconds and more preferably from 30 seconds to 70 seconds. This makes it possible to suitably measure the hemoglobin variants such as HbA0, HbE, HbA2, and HbD and to separate each of the hemoglobin variants with high accuracy according to the measurement time.

The time to supply the eluent O is preferably from 14 seconds to 71 seconds and more preferably from 20 seconds to 47 seconds. This makes it possible to suitably measure HbBart's. In addition, the time to supply the eluent M is preferably from 18 seconds to 95 seconds and more preferably 27 seconds to 63 seconds. This makes it possible to suitably measure hemoglobin such as HbC and HbCS.

As the method of measuring hemoglobin variants according to an aspect of the invention, it is preferable to supply at least the eluent L. It is possible to collectively measure, for example, at least one of the hemoglobin variants such as HbBart's, HbH, HbF, HbA0, HbE, HbA2, HbD, and HbS by supplying the eluent L singly or in combination with other eluents and using it in the measurement of hemoglobin variants. By using the eluent L singly, it is possible to collectively measure hemoglobin variants such as HbA0, HbE, HbA2, and HbD in particular, and it is useful for the measurement of at least one kind of hemoglobin of, for example, normal hemoglobin, abnormal hemoglobin, or hemoglobin that is a marker for thalassemia. Furthermore, it is more preferable to supply the eluent O or the eluent M of another eluent in combination with the eluent L. For example, it is possible to measure hemoglobin variants such as HbH, HbF, and HbA1c by supplying the eluent L and eluent O. In addition, it is possible to measure HbS, for example, by supplying the eluent L and the eluent M. Incidentally, Hb that is eluted before Hb of the target to be measured can be eluted by another eluent, if necessary.

In addition, as the method of measuring hemoglobin variants according to an aspect of the invention, it is preferable to supply a mixed liquid prepared by mixing at least one of the eluent O or the eluent M with the eluent L.

Among them, a mixed liquid prepared by mixing the eluent L and the eluent O is useful when collectively measuring HbA1c and HbF, and HbH of a thalassemia marker, and a mixed liquid prepared by mixing the eluent L and the eluent M is useful in measuring abnormal Hb such as HbS.

As the method of measuring hemoglobin variants according to an aspect of the invention, it is more preferable to supply the eluent L, a mixed liquid prepared by mixing the eluent L and the eluent O, and a mixed liquid prepared by mixing the eluent M and the eluent L in combination. This makes it possible to collectively measure HbA1c, HbF, HbA0, HbA2, HbH of a thalassemia marker, and a number of abnormal Hb. Examples of the measurable abnormal Hb include HbE, HbS, and HbD.

In an aspect of the invention, HbA1c and abnormal Hb can be collectively measured, and thus the diabetes diagnosis and the identification of at least one kind of hemoglobin of abnormal hemoglobin, or hemoglobin that is a marker for thalassemia can be realized by a single measurement apparatus. Hence, it is possible to cut down the cost as compared with a case in which the measurement of HbA1c and the measurement of at least one kind of hemoglobin of abnormal hemoglobin, or hemoglobin that is a marker for thalassemia are separately conducted. In addition, a highly accurate measurement result of HbA1c can be obtained in the case of measuring a sample containing at least one kind of hemoglobin of abnormal hemoglobin, or hemoglobin that is a marker for thalassemia. Furthermore, it is possible to confirm the presence or absence of at least one kind of hemoglobin of abnormal hemoglobin, or hemoglobin that is a marker for thalassemia at the time of measuring HbA1c, and the results can be utilized in the medical field and for the treatment, test, and research in the clinical laboratory test field.

In the mixed liquid prepared by mixing the eluent L and the eluent O, the mixing ratio of the eluent L to the eluent O may be adjusted, if appropriate, depending on the hemoglobin of the target to be measured. For example, in a case in which hemoglobin variants such as HbF, and HbA1c are the target to be measured, the mixing ratio of eluent L : eluent O is preferably from 1:2 to 1:4 and more preferably from 1:2.5 to 1:3.5.

The time to supply the mixed liquid prepared by mixing the eluent L and the eluent O is preferably from 11 seconds to 64 seconds and more preferably from 16 seconds to 39 seconds. This makes it possible to suitably measure HbH, HbA1c, and HbF. In more detail, it is possible to separate each of the measurable hemoglobin variants such as HbH, HbA1c, and HbF with high accuracy according to the measurement time, for example, as the elution times of the hemoglobin variants from the separation column do not overlap with one another.

In addition, for the mixed liquid prepared by mixing the eluent M and the eluent L, the mixing ratio of the eluent M to the eluent L may be adjusted, if appropriate, depending on the hemoglobin of the target to be measured. For example, in a case in which a hemoglobin variant such as HbS is the target to be measured, the mixing ratio of eluent M : eluent L is preferably from 1 : 2 to 1 : 4 and more preferably from 1:2.5 to 1:3.5.

The time to supply the mixed liquid prepared by mixing the eluent M and the eluent L is preferably from 9 seconds to 47 seconds and more preferably from 13 seconds to 32 seconds. This makes it possible to suitably measure hemoglobin such as HbS.

In addition to these, in the method of measuring hemoglobin variants according to an aspect of the invention, it is preferable to supply at least one liquid selected from the group consisting of the eluent M, the eluent O, and a mixed liquid prepared by mixing the eluent B and the eluent A. This makes it possible to measure at least one of HbBart's, HbH, HbF, HbA1c, HbA0, HbA2, HbE, HbD, HbS, HbC, or HbConstantSpring, and in particular, it is possible to measure at least one of HbBart's, HbS, HbC, or HbCS.

Among them, it is more preferable to measure the hemoglobin variants by supplying the eluent M and the eluent O successively or to measure the hemoglobin variants by supplying a mixed liquid prepared by mixing the eluent B and the eluent A and the eluent O successively. It is possible to measure at least one of HbBart's, HbH, HbF, HbA1c, HbA0, HbA2, HbE, HbD, HbS, HbC, or HbConstantSpring, and in particular, it is possible to collectively measure all of HbBart's, HbC and HbCS by supplying the eluent M and the eluent O successively. It is possible to measure at least one of HbBart's, HbH, HbF, HbA1c, HbA0, HbA2, HbE, HbD, HbS, HbC, or HbConstantSpring, and in particular, it is possible to collectively measure all of HbBart's, HbS, HbC, and HbCS by supplying a mixed liquid prepared by mixing the eluent B and the eluent A and the eluent O successively.

In addition, in the mixed liquid prepared by mixing the eluent B and the eluent A, the mixing ratio of the eluent B to the eluent A may be adjusted, if appropriate, depending on the hemoglobin of the target to be measured. For example, in a case in which hemoglobin variants such as HbS, HbC, and HbCS are the target to be measured, the mixing ratio of eluent B : eluent A is preferably from 1:2 to 1:4 and more preferably from 1:2.5 to 1:3.5.

The time to supply the mixed liquid prepared by mixing the eluent B and the eluent A is preferably from 17 seconds to 86 seconds and more preferably from 24 seconds to 57 seconds. This makes it possible to suitably measure hemoglobin such as HbS, HbC, and HbCS.

In a case in which a plurality of eluents are supplied, a plurality of mixed liquids are supplied, or eluents and mixed liquids are supplied in combination, the order (supplying sequence) of supplying the eluents and the mixed liquids is not particularly limited, and the eluents and the mixed liquids may be supplied in an arbitrary order. Examples of the supplying sequence include the following supplying sequence 1 to supplying sequence 5.

(Supplying Sequence 1)

In the case of supplying the eluent L, the eluent O and the eluent M as the eluent, the order of supplying is not limited as described above, but it is preferable to supply them in the order of the eluent O, the eluent L, and the eluent M. This makes it possible to measure at least one of the hemoglobin variants such as HbBart's, HbH, HbF, HbA1c, HbA0, HbE, HbA2, HbD, HbS, HbC, and HbCS, and in particular, it is possible to collectively measure the hemoglobin variants such as HbBart's, HbA0, HbE, HbA2, HbD, HbC, and HbCS. In addition, the measurable hemoglobin variants can be separated into each hemoglobin variant with high accuracy according to the measurement time.

In addition, for example, in the case of separating HbA0, HbE, and HbD or separating HbA0, HbA2, and HbD by supplying the eluent L, it is possible to separate the respective hemoglobin variants with high accuracy according to the measurement time, for example, as the elution times of these hemoglobin variants from the separation column do not overlap with one another. Furthermore, for example, in the case of separating HbC and HbCS by supplying the eluent M, it is possible to separate the respective hemoglobin variants with high accuracy according to the measurement time, for example, as the elution times of these hemoglobin variants from the separation column do not overlap with each other.

(Supplying Sequence 2)

In the case of supplying (1) the eluent O and (2) the eluent L in this order, a mixed liquid prepared by mixing the eluent L and the eluent O may be supplied between (1) and (2). It is possible to collectively measure HbH, HbA1c and HbF by supplying the mixed liquid prepared by mixing the eluent L and the eluent O. Hence, it is possible to measure at least one of HbBart's, HbH, HbF, HbA1c, HbA0, HbA2, HbE, or HbD, and it is possible to separate the measurable hemoglobin variants with high accuracy according to the measurement time, for example, as the elution times of the respective hemoglobin variants from the separation column do not overlap with one another.

(Supplying Sequence 3)

Next, in the case of supplying (1) the eluent L and (2) the eluent M in this order, a mixed liquid prepared by mixing the eluent M and the eluent L may be supplied between (1) and (2). It is possible to measure abnormal Hb such as HbS by supplying the mixed liquid prepared by mixing the eluent M and the eluent L. Hence, it is possible to measure at least one of HbA0, HbA2, HbE, HbD, HbS, HbC, or HbConstantSpring, and it is possible to separate the measurable hemoglobin variants with high accuracy according to the measurement time, for example, as the elution times of the respective hemoglobin variants from the separation column do not overlap with one another.

(Supplying Sequence 4)

It is preferable to supply the eluent O, the mixed liquid prepared by mixing the eluent L and the eluent O, the eluent L, the mixed liquid prepared by mixing the eluent M and the eluent L, and the eluent M in this order. By virtue of this, the collective measurement of hemoglobin variants such as HbBart's, HbH, HbF, HbA1c, HbA0, HbE, HbA2, HbD, HbS, HbC, and HbCS is possible. It is possible to separate the measurable hemoglobin variants into each hemoglobin variant with high accuracy according to the measurement time.
In addition, for example, in the case of separating HbH, HbA1c, and HbF by supplying the mixed liquid prepared by mixing the eluent L and the eluent O, each of the hemoglobin variants can be separated with high accuracy according to the measurement time, for example, as the elution times of these hemoglobin variants from the separation column do not overlap with one another.

(Supplying Sequence 5)

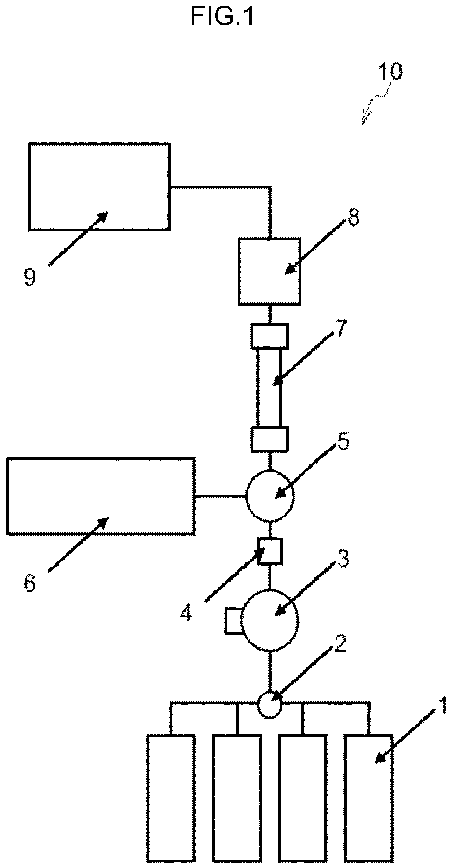

Moreover, it is preferable to supply (1) the eluent O, (2) the eluent A, and (3) the eluent L in this order. This makes it possible to collectively measure HbBart's, HbH, HbF, HbA1c, HbA0, HbE, HbA2, and HbD. For example, it is possible to separate the measurable hemoglobin variants with high accuracy according to the measurement time, for example, as the elution times of the respective hemoglobin variants from the separation column do not overlap with one another.
The supplying sequences other than the supplying sequences 1 to 5 described above are also included in the scope of the invention in the case of using the eluent L, the eluent O, the eluent M, the eluent A or the eluent B.
Hereinafter, the configuration of the measurement apparatus and the operation flow of the measurement apparatus according to an aspect of the invention will be described with reference to Fig. 1. Fig. 1 illustrates the measurement apparatus according to an embodiment of an aspect of the invention. A measurement apparatus 10 is an apparatus for measuring the hemoglobin variants contained in a measurement sample, and it is equipped with an eluent tank 1, a switching valve 2, a supply pump 3, a pressure gauge 4, an injection valve 5, an auto-sampler 6, a separation column 7, a detector 8, and a waste liquor container 9.
Incidentally, the method of measuring hemoglobin variants described above can be implemented using a measurement apparatus to be described below.
The eluent tank 1 is a tank for storing each of the eluents described above. A plurality of eluent tanks 1 are provided, and each of the eluents described above is stored in each of the eluent tanks 1. The eluent stored in the eluent tank 1 is supplied to the switching valve 2 and the supply pump 3 via a flow path.
It is possible to change the eluent to be supplied by switching the switching valve 2. For example, the switching valve 2 is driven on the basis of the supplying sequence set and thus the switching valve 2 is switched, and supplying of the eluent in accordance with the sequence is conducted.

It is possible to supply an eluent with constant flow to the injection valve 5 by the supply pump 3, and it is possible to confirm that the eluent with constant flow is supplied without fluctuation in pressure by the pressure gauge 4. In addition, it is possible to mix the respective eluents in the flow path by continuously switching the switching valve 2 when the eluents are supplied by the supply pump 3.

By the auto-sampler 6, the measurement sample passes through the injection valve 5 and is injected into the separation column 7. Furthermore, the eluent also passes through the injection valve 5 and is injected into the separation column 7. Thereafter, the separation of the hemoglobin variants is conducted in the inside of the separation column 7 by the eluent supplied, the hemoglobin variants are eluted in accordance with the kind of the eluent supplied and emerge from the separation column 7.

The hemoglobin variant emerged is detected by the detector 8 such as an ultraviolet or visible spectrophotometer. The chromatogram is drawn in accordance with the detection result by the detector 8 and displayed on a display device (not illustrated). After detection by the detector 8, the liquid containing the hemoglobin variant is injected into the waste liquor container 9 as the waste liquor.

The measurement apparatus for measuring hemoglobin variants is not particularly limited, and it is possible to use a commercially available high-performance liquid chromatographic system.

Examples of the separation column include a cation exchange column. The cation exchange column preferably contains, for example, at least any of a sulfo group, a carboxyl group, or a phosphoric acid group as a cation exchange group, and more preferably contains a sulfo group as a cation exchange group.

For the separation column, those which are filled with a column packing material of a column base may be used. Examples of the column packing material include a polymer gel such as a methacrylic acid-methacrylic acid ester copolymer or an acrylic acid-acrylic acid ester copolymer, or an inorganic gel such as silica gel. Among them, a methacrylic acid-methacrylic acid ester copolymer is preferable.

The length of the separation column is, for example, preferably 1 mm or more, more preferably 5 mm or more, and still more preferably 10 mm or more. In addition, the length of the separation column is, for example, preferably 300 mm or less, more preferably 200 mm or less, still more preferably 150 mm or less, still more preferably 100 mm or less, still more preferably 75 mm or less, still more preferably 50 mm or less, still more preferably 40 mm or less, still more preferably 35 mm or less, and still more preferably 30 mm or less.

The inner diameter ($\phi$) of the separation column is, for example, preferably 0.1 mm or more, more preferably 0.2 mm or more, still more preferably 0.3 mm or more, still more preferably 0.5 mm or more, still more preferably 1 mm or more, still more preferably 2 mm or more, and still more preferably 3 mm or more. In addition, the internal diameter of the separation column is, for example, preferably 50 mm or less, more preferably 30 mm or less, still more preferably 20 mm or less, still more preferably 10 mm or less, and still more preferably 5 mm or less.

The average particle size of the column packing material in the separation column is, for example, preferably 0.1 $\mu$m or more, more preferably 0.5 $\mu$m or more, still more preferably 1 $\mu$m or more, and particularly preferably 3 $\mu$m or more. In addition, the average particle size of the column packing material in the separation column is, for example, preferably 50 $\mu$m or less, more preferably 20 $\mu$m or less, and still more preferably 10 $\mu$m or less.

The coefficient value of variation (CV value) of the average particle size of the column packing material in the separation column is not particularly limited, and it is preferably 40% or less, more preferably 30% or less, and still more preferably 15% or less. Incidentally, the CV value means a numerical value determined by the following Formula.

$$\text{CV value } (\%) = (\text{standard deviation of particle size/average particle size}) \times 100$$

In addition, it is preferable that the measurement apparatus for measuring hemoglobin variants has one supply pump. It is possible to decrease the size and cost of the measurement apparatus when a configuration is adopted in which the respective eluents or the mixed liquid prepared by mixing the respective eluents are supplied to the separation column successively by one supply pump. Incidentally, the measurement apparatus for measuring hemoglobin variants may adopt a gradient method in which two or more supply pumps are provided, a tank containing one eluent is connected to one supply pump, and the eluents are supplied to the separation column while changing the mixing ratio of two or more kinds of eluents.

Examples

Next, the results obtained by measuring respective hemoglobin variants by the measurement method of an aspect of the invention will be described in accordance with the following Examples, but the invention is not limited to Examples. Incidentally, in the following description, the "%" means "% by mass" in all cases unless otherwise specified.

[Measurement Conditions]

In the following Examples and Comparative Examples, the measurement of hemoglobin variants was conducted in accordance with the following measurement conditions.

- Liquid chromatographic system: glycosylated hemoglobin analyzer (manufactured by ARKRAY, Inc.)
- Separation column: $\phi 4.6 \times 20$ mm gel (column packing material): methacrylic acid-methacrylic acid ester copolymer
- Flow rate: 2.1 ml/min
- Elution condition: low pressure step gradient by single pump

[Example 1]

In the method of measuring hemoglobin variants of Example 1, the eluent L, the eluent O, the eluent M and the eluent B were used singly or by mixing at a mixing ratio as presented in the following supplying sequence. The measurement sample and the eluent B used in the present Example are as presented below, and the eluent L, the eluent O, and the eluent M are as presented in the following Table 1.

- Measurement sample: (1) healthy individual specimen, (2) specimen containing HbBart's, HbF, HbA0, HbE, HbD, HbS, HbC and HbCS (blood specimen prepared by mixing blood samples), (3) specimen containing HbBart's and HbH

- Eluent B: pH of 8.05 and osmotic pressure of 547 mOsm

[Table 1]

| | Component 1 Dipotassium hydrogen phosphate | Component 2 Potassium dihydrogen phosphate | Component 2/component 1 | Osmotic pressure (mOsm) | pH | Sodium azide | Sodium propionate | 2-phenoxyethanol | Distilled water |
|---|---|---|---|---|---|---|---|---|---|
| Eluent L | 0.25% (w/v) | 0.65% (w/v) | 2.6 | 133 | 6.35 | 0.0182% | 0.01% | 0.05% | 99.02% |
| Eluent O | 0.03% (w/v) | 1.46% (w/v) | 48.7 | 206 | 5.18 | 0.017% | 0.01% | 0.05% | 98.44% |
| Eluent M | 0.69% (w/v) | 0.61% (w/v) | 0.9 | 185 | 6.76 | 0.0174% | 0.01% | 0.05% | 98.63% |

The hemoglobin variants were measured using the specimens of (1) to (3) as the measurement sample and by supplying the respective eluents and the respective mixed liquids to the separation column in the following order of (I) to (VII).

- Supplying sequence in the case of using the specimen of (1) as the measurement sample: (I) eluent O is supplied for 1 second, (II) mixed liquid prepared by mixing eluent L and eluent O at a mixing ratio of 1:3 is supplied for 64 seconds, (III) eluent L is supplied for 49 seconds, (IV) mixed liquid prepared by mixing eluent M and eluent L at a mixing ratio of 1:3 is supplied for 22 seconds, (V) eluent M is supplied for 15 seconds, (VI) eluent B is supplied for 4 seconds, and (VII) eluent O is supplied for 15 seconds.
- Supplying sequence in the case of using the specimen of (2) as the measurement sample: (I) eluent O is supplied for 1 second, (II) mixed liquid prepared by mixing eluent L and eluent O at a mixing ratio of 1:3 is supplied for 32 seconds, (III) eluent L is supplied for 49 seconds, (IV) mixed liquid prepared by mixing eluent M and eluent L at a mixing ratio of 1:3 is supplied for 22 seconds, (V) eluent M is supplied for 55 seconds, (VI) eluent B is supplied for 4 seconds, and (VII) eluent O is supplied for 15 seconds.
- Supplying sequence in the case of using the specimen of (3) as the measurement sample: (I) eluent O is supplied for 1 second, (II) mixed liquid prepared by mixing eluent L and eluent O at a mixing ratio of 1:3 is supplied for 32 seconds, (III) eluent L is supplied for 49 seconds, (IV) mixed liquid prepared by mixing eluent M and eluent L at a mixing ratio of 1:3 is supplied for 22 seconds, (V) eluent M is supplied for 15 seconds, (VI) eluent B is supplied for 4 seconds, and (VII) eluent O is supplied for 15 seconds.

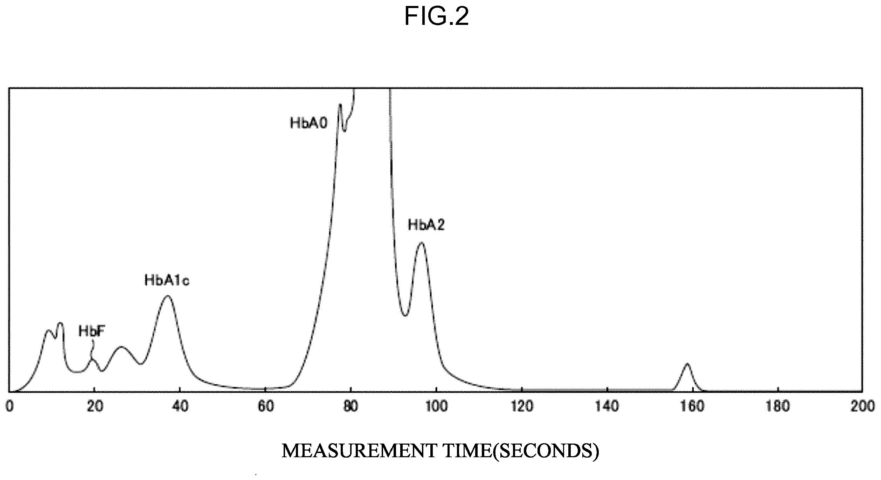

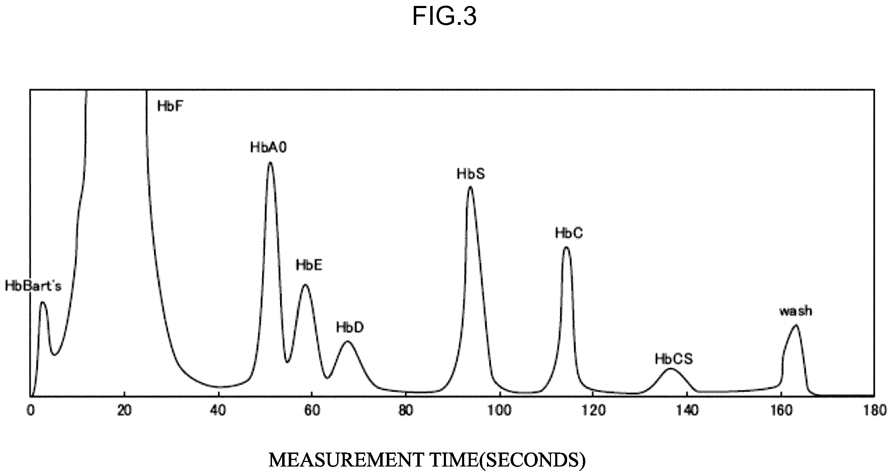

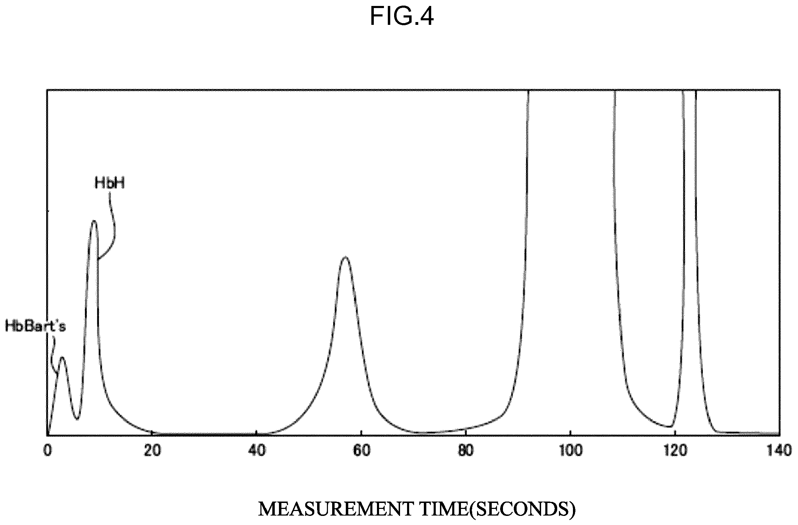

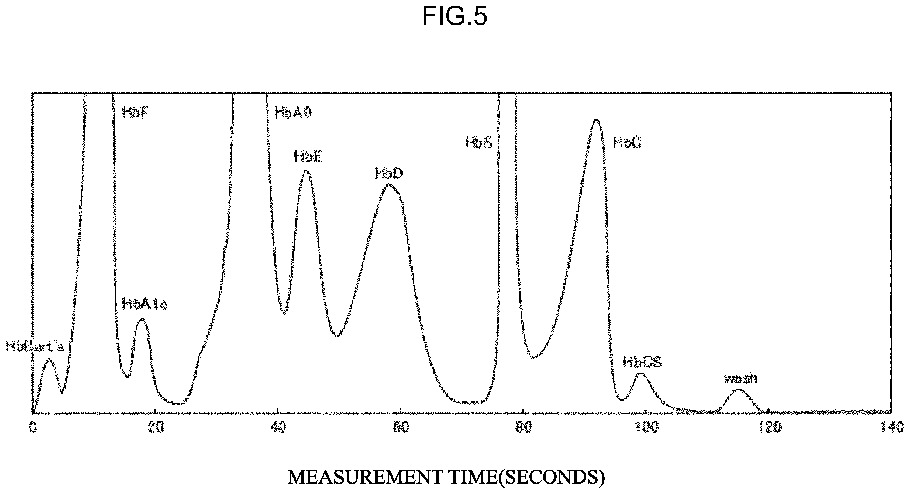

The measurement results of Example 1 are illustrated in Fig. 2 to Fig. 4. Fig. 2 illustrates the measurement results in the case of using (1) healthy individual specimen as the measurement sample, Fig. 3 illustrates the measurement results in the case of using (2) specimen containing HbBart's, HbF, HbA0, HbE, HbD, HbS, HbC and HbCS (blood specimen prepared by mixing blood samples) as the measurement sample, and Fig. 4 illustrates the measurement results in the case of using (3) specimen containing HbBart's and HbH as the measurement sample. In all of Fig. 2 to Fig. 4, the vertical axis represents the absorbance, and the horizontal axis represents the measurement time (seconds) (hereinafter, the same applies to Fig. 5 to Fig. 11). From Fig. 2 to Fig. 4, it can be seen what hemoglobin variant is measurable in the case of supplying any of the eluent or the mixed liquid.

As illustrated in Fig. 2, HbA1c was measured after HbF was measured by (II) above, and HbA2 was measured after HbA0 was measured by (III) above. Consequently, it has been indicated that HbA1c and other Hb can be collectively separated by the above supplying sequence.

Next, as illustrated in Fig. 3, HbBart's was measured by (I) above, HbF was measured by (II) above, the hemoglobin variants were measured in the order of HbA0, HbE, and HbD by (III) above, HbS was measured by (IV) above, and HbCS was measured after HbC was measured by (V) above. Consequently, it has been indicated that abnormal Hb, and HbBart's and HbCS which are markers for thalassemia can be collectively separated by the above supplying sequence.

In addition, as illustrated in Fig. 4, HbBart's was measured by (I) above, and HbH was measured by (II) above.

[Example 2]

In the method of measuring hemoglobin variants of Example 2, the eluent L, the eluent O, the eluent A and the eluent B were used singly or by mixing at a mixing ratio as presented in the following supplying sequence. The measurement sample, and the eluent A and the eluent B used in the present Example are as presented below, and the eluent L and the eluent O are as presented in the following Table 2.

- Measurement sample: specimen containing HbBart's, HbF, HbA1c, HbA0, HbE, HbD, HbS, HbC and HbCS (blood specimen prepared by mixing blood samples)
- Eluent A: pH of 5.35 and osmotic pressure of 207 mOsm
- Eluent B: pH of 8.05 and osmotic pressure of 469 mOsm

[Table 2]

|  | Component 1 | Component 2 | Component 2/component 1 | Osmotic pressure (mOsm) | pH | Sodium azide | Sodium propionate | 2-phenoxyethanol | Distilled water |
|---|---|---|---|---|---|---|---|---|---|
|  | Dipotassium hydrogen phosphate | Potassium dihydrogen phosphate | | | | | | | |
| Etuent L | 0.24% (w/v) | 0.66% (w/v) | 2.75 | 134 | 6.30 | 0.0182% | 0.01% | 0.05% | 99.02% |
| Eluent O | 0.03% (w/v) | 1.46% (w/v) | 48.7 | 206 | 5.18 | 0.017% | 0.01% | 0.05% | 98.44% |

The hemoglobin variants were measured using the specimens as the measurement sample and by supplying the respective eluents and the respective mixed liquids to the separation column in the following order of (I) to (VI).

- Supplying sequence: the hemoglobin variants were measured by supplying the respective eluents to the separation column in the following order of (I) to (VI). (I) eluent O is supplied for 1 second, (II) eluent A is supplied for 20 seconds, (III) eluent L is supplied for 50 seconds, (IV) mixed liquid prepared by mixing eluent B and eluent A at a mixing ratio of 1:3 is supplied for 40 seconds, (V) eluent B is supplied for 8 seconds, and (VI) eluent O is supplied for 15 seconds.

The measurement results of Example 2 are illustrated in Fig. 5. Fig. 5 illustrates the measurement results in the case of using the specimen containing HbBart's, HbF, HbA1c, HbA0, HbE, HbD, HbS, HbC and HbCS as the measurement sample. As illustrated in Fig. 5, it has been indicated that HbA1c, abnormal Hb, HbBart's and the like can be collectively separated by the above supplying sequence.

[Examples 3 and 4]

In the method of measuring hemoglobin variants of Example 3, the eluent L, the eluent O, the eluent M and the eluent B were used singly or by mixing at a mixing ratio as presented in the following supplying sequence. In the method of measuring hemoglobin variants of Example 4, comparative eluent L was used instead of the eluent L and the eluent L in the supplying sequence below was changed to the comparative eluent L. The measurement sample and the eluent B used in these Examples are as presented below, and the eluent L, the eluent O, the eluent M and the comparative eluent L are as presented in the following Table 3.

- Measurement sample: (1) specimen containing HbE, (2) healthy individual specimen

- Eluent B: pH of 8.05 and osmotic pressure of 547 mOsm

[Table 3]

| | Component 1 | Component 2 | Component 2/component 1 | Osmotic pressure (mOsm) | pH | Sodium azide | Sodium propionate | 2-phenoxyethanol | Distilled water |
|---|---|---|---|---|---|---|---|---|---|
| | Dipotassium hydrogen phosphate | Potassium dihydrogen phosphate | | | | | | | |
| Eluent L | 0.25% (w/v) | 0.65% (w/v) | 2.6 | 133 | 6.35 | 0.0182% | 0.01% | 0.05% | 99.02% |
| Eluent O | 0.03% (w/v) | 1.46% (w/v) | 48.7 | 206 | 5.18 | 0.017% | 0.01% | 0.05% | 98.44% |
| Eluent M | 0.69% (w/v) | 0.61% (w/v) | 0.9 | 185 | 6.76 | 0.0174% | 0.01% | 0.05% | 98.63% |
| Comparative eluent L | 0.73% (w/v) | 0.18% (w/v) | 0.25 | 133 | 7.45 | 0.0182% | 0.01% | 0.05% | 99.02% |

The hemoglobin variants were measured using each of the specimens as the measurement sample and by supplying the respective eluents and the respective mixed liquids to the separation column in the following order of (I) to (VII).

- Supplying sequence: the hemoglobin variants were measured by supplying the respective eluents to the separation column in the following order of (I) to (VII). (I) eluent O is supplied for 1 second, (II) mixed liquid prepared by mixing eluent L and eluent O at a mixing ratio of 1:3 is supplied for 64 seconds, (III) eluent L is supplied for 49 seconds, (IV) mixed liquid prepared by mixing eluent M and eluent L at a mixing ratio of 1:3 is supplied for 22 seconds, (V) eluent M is supplied for 15 seconds, (VI) eluent B is supplied for 4 seconds, and (VII) eluent O is supplied for 15 seconds.

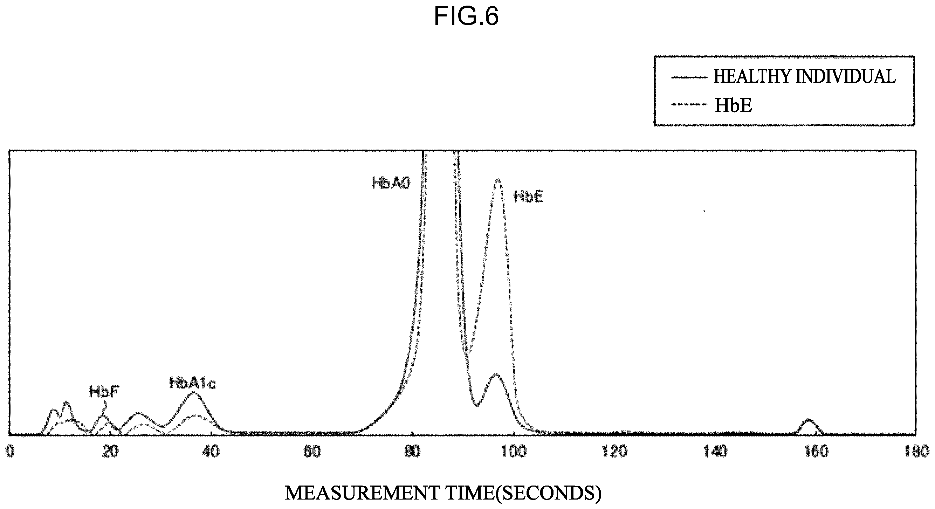

The measurement results of Example 3 are illustrated in Fig. 6. Fig. 6 illustrates the measurement results in the case of using the specimen containing HbE and the healthy individual specimen as the measurement sample. As illustrated in Fig. 6, it has been indicated that HbA1c and abnormal Hb can be collectively separated by the above supplying sequence.

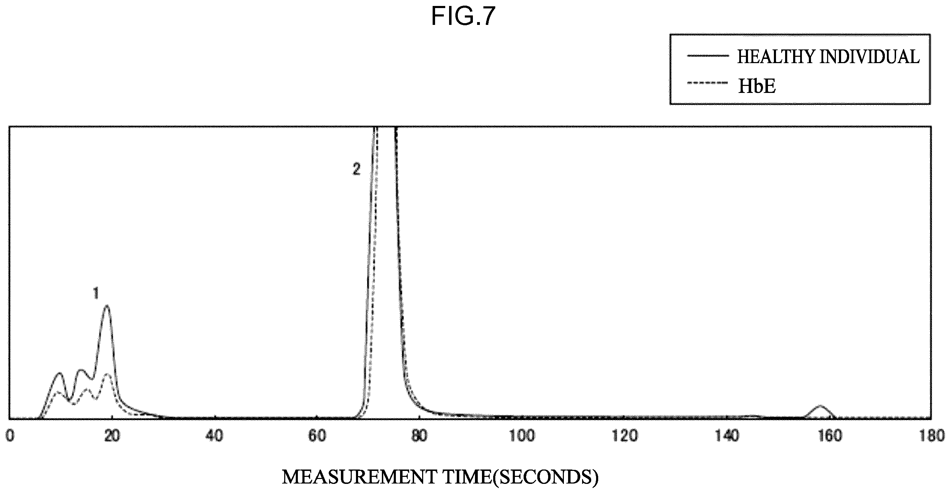

The measurement results of Example 4 are illustrated in Fig. 7. Fig. 7 illustrates the measurement results in the case of using the specimen containing HbE and the healthy individual specimen as the measurement sample and the comparative eluent L instead of the eluent L. As illustrated in Fig. 7, in the above supplying sequence, the elution positions of HbA1c and HbF (peak 1) overlapped with each other and also the elution positions of HbA0 and HbE (peak 2) overlapped with each other.

From the results of Fig. 6 and Fig. 7, it can be seen that the elution positions of HbA1c and HbF overlapped with each other, and the elution positions of HbA0 and HbE overlapped with each other in the case of using the comparative eluent L, but the elution positions of these hemoglobin variants do not overlap with each other in the case of using the eluent L. Consequently, it is possible to separate more hemoglobin variants with high accuracy according to the measurement time by using the eluent L.

[Examples 5 and 6]

In the method of measuring hemoglobin variants of Example 5, the eluent L, the eluent O, the eluent M and the eluent B were used singly or by mixing at a mixing ratio as presented in the following supplying sequence. In the method of measuring hemoglobin variants of Example 6, comparative eluent O was used instead of the eluent O and the eluent O in the supplying sequence below was changed to the comparative eluent O. The measurement sample and the eluent B used in these Examples are as presented below, and the eluent L, the eluent O, the eluent M and the comparative eluent O are as presented in the following Table 4.

- Measurement sample: specimen containing HbBart's
- Eluent B: pH of 8.05 and osmotic pressure of 547 mOsm

[Table 4]

| | Component 1 | Component 2 | Component 2/component 1 | Osmotic pressure (mOsm) | pH | Sodium azide | Sodium propionate | 2-phenoxyethanol | Distilled water |
| | Dipotassium hydrogen phosphate | Potassium dihydrogen phosphate | | | | | | | |
|---|---|---|---|---|---|---|---|---|---|
| Eluent L | 0.25% (w/v) | 0.65% (w/v) | 2.6 | 133 | 6.35 | 0.0182% | 0.01% | 0.05% | 99.02% |
| Eluent O | 0.03% (w/v) | 1.46% (w/v) | 48.7 | 206 | 5.18 | 0.017% | 0.01% | 0.05% | 98.44% |
| Eluent M | 0.69% (w/v) | 0.61% (w/v) | 0.9 | 185 | 6.76 | 0.0174% | 0.01% | 0.05% | 98.63% |
| Comparative eluent O | 0.07% (w/v) | 1.40% (w/v) | 20.0 | 207 | 5.46 | 0.017% | 0.01% | 0.05% | 98.44 |

The hemoglobin variants were measured using the specimen as the measurement sample and by supplying the respective eluents and the respective mixed liquids to the separation column in the following order of (I) to (VII).

• Supplying sequence: the hemoglobin variants were measured by supplying the respective eluents to the separation column in the following order of (I) to (VII). (I) eluent O is supplied for 1 second, (II) mixed liquid prepared by mixing eluent L and eluent O at a mixing ratio of 1:3 is supplied for 32 seconds, (III) eluent L is supplied for 49 seconds, (IV) mixed liquid prepared by mixing eluent M and eluent L at a mixing ratio of 1:3 is supplied for 22 seconds, (V) eluent M is supplied for 15 seconds, (VI) eluent B is supplied for 4 seconds, and (VII) eluent O is supplied for 15 seconds.

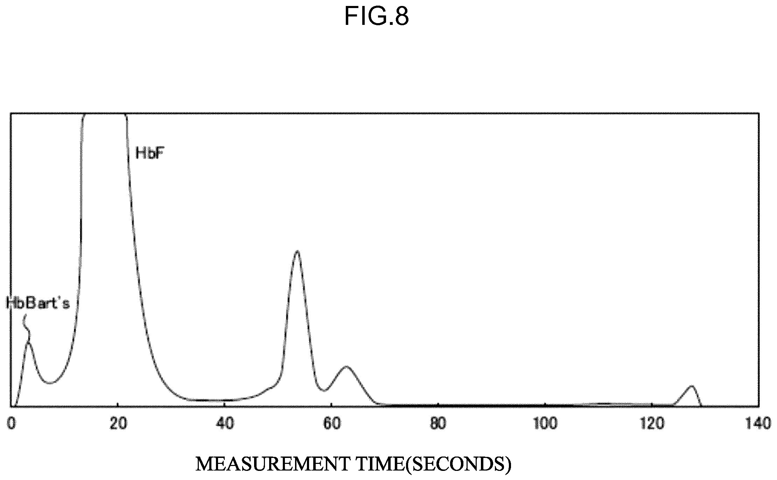

The measurement results of Example 5 are illustrated in Fig. 8. Fig. 8 illustrates the measurement results in the case of using the specimen containing HbBart's as the measurement sample. As illustrated in Fig. 8, it has been indicated that HbBart's and HbF can be collectively separated by the above supplying sequence.

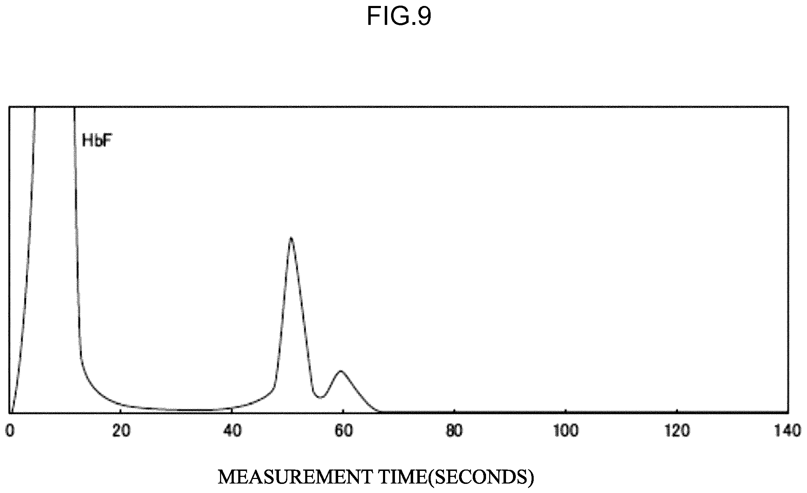

The measurement results of Example 6 are illustrated in Fig. 9. Fig. 9 illustrates the measurement results in the case of using the specimen containing HbBart's as the measurement sample and the comparative eluent O instead of the eluent O. As illustrated in Fig. 9, in the above supplying sequence, the elution positions of HbBart's and HbF overlapped with each other.

From the results of Fig. 8 and Fig. 9, it can be seen that the elution positions of HbBart's and HbF overlapped with each other in the case of using the comparative eluent O, but the elution positions of HbBart's and HbF do not overlap with each other in the case of using the eluent O. Consequently, it is possible to separate more hemoglobin variants with high accuracy according to the measurement time by using the eluent O.

[Examples 7 and 8]

In the method of measuring hemoglobin variants of Example 7, the eluent L, the eluent O, the eluent M and the eluent B were used singly or by mixing at a mixing ratio as presented in the following supplying sequence. In the method of measuring hemoglobin variants of Example 8, comparative eluent M was used instead of the eluent M and the eluent M in the supplying sequence below was changed to the comparative eluent M. The measurement sample and the eluent B used in these Examples are as presented below, and the eluent L, the eluent O, the eluent M and the comparative eluent M are as presented in the following Table 5.

• Measurement sample: (1) specimen containing HbS and HbC, (2) specimen containing HbCS

• Eluent B: pH of 8.05 and osmotic pressure of 547 mOsm

[Table 5]

| | Component 1 | Component 2 | Component 2/component 1 | Osmotic pressure (mOsm) | pH | Sodium azide | Sodium propionate | 2-phenoxyethanol | Distilled water |
|---|---|---|---|---|---|---|---|---|---|
| | Dipotassium hydrogen phosphate | Potassium dihydrogen phosphate | | | | | | | |
| Eluent L | 0.25% (w/v) | 0.65% (w/v) | 2.6 | 133 | 6.35 | 0.0182% | 0.01% | 0.05% | 99.02% |
| Eluent O | 0.03% (w/v) | 1.46% (w-v) | 48.7 | 206 | 5.18 | 0.017% | 0.01% | 0.05% | 98.44% |
| Eluent M | 0.69% (w/v) | 0.61% (w/v) | 0.9 | 185 | 6.76 | 0.0174% | 0.01% | 0.05% | 98.63% |
| Comparative eluent M | 1.04% (w/v) | 0.26% (w/v) | 0.25 | 188 | 7.33 | 0.0174% | 0.01% | 0.05% | 98.63% |

The hemoglobin variants were measured using the specimen as the measurement sample and by supplying the respective eluents and the respective mixed liquids to the separation column in the following order of (I) to (VII).

- Supplying sequence: the hemoglobin variants were measured by supplying the respective eluents to the separation column in the following order of (I) to (VII). (I) eluent O is supplied for 1 second, (II) mixed liquid prepared by mixing eluent L and eluent O at a mixing ratio of 1:3 is supplied for 32 seconds, (III) eluent L is supplied for 49 seconds, (IV) mixed liquid prepared by mixing eluent M and eluent L at a mixing ratio of 1:3 is supplied for 22 seconds, (V) eluent M is supplied for 55 seconds, (VI) eluent B is supplied for 4 seconds, and (VII) eluent O is supplied for 15 seconds.

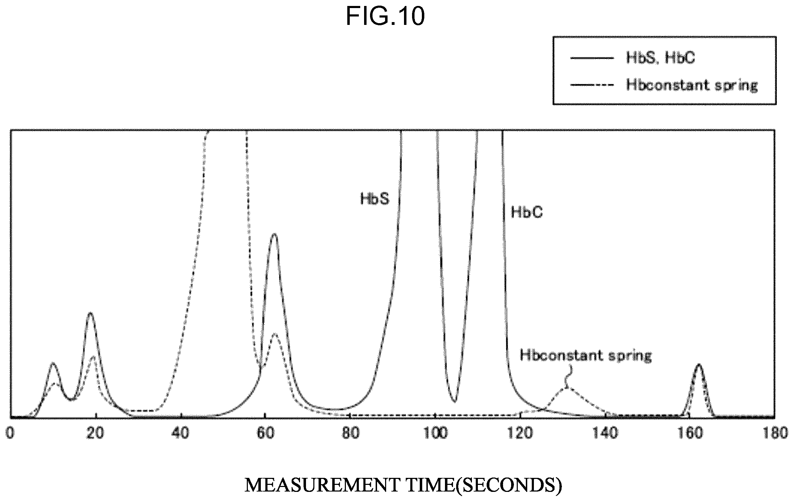

The measurement results of Example 7 are illustrated in Fig. 10. Fig. 10 illustrates the measurement results in the case of using (1) specimen containing HbS and HbC and (2) specimen containing HbCS as the measurement sample. As illustrated in Fig. 10, it was possible to collectively measure HbS and HbC and to measure HbCS at an elution position different from those of HbS and HbC by the above supplying sequence.

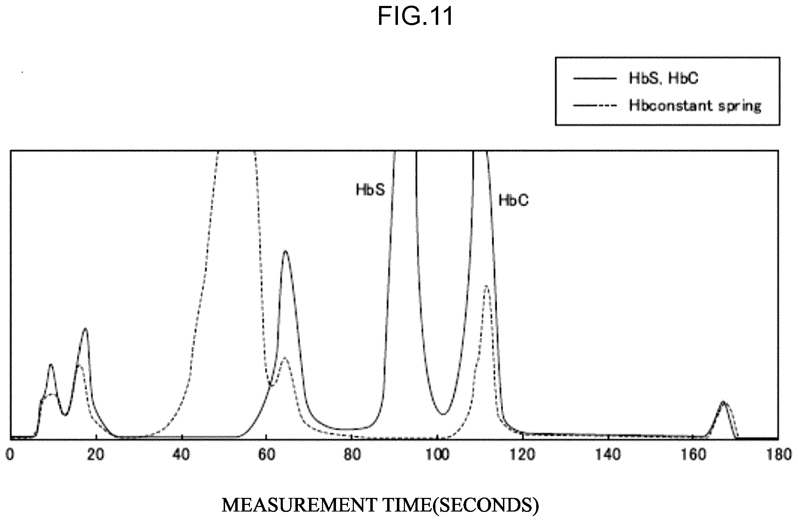

The measurement results of Example 8 are illustrated in Fig. 11. Fig. 11 illustrates the measurement results in the case of using (1) specimen containing HbS and HbC and (2) specimen containing HbCS as the measurement sample and the comparative eluent M instead of the eluent M. As illustrated in Fig. 11, in the above supplying sequence, the elution positions of HbC and HbCS overlapped with each other but it was possible to collectively measure HbS and HbC.

Next, the results of experiments to determine preferred ranges of the ratio of component 2/component 1, the osmotic pressure, and the pH in each eluent by changing the content rates of the component 1 and the component 2 are presented.

[Experiment 1]

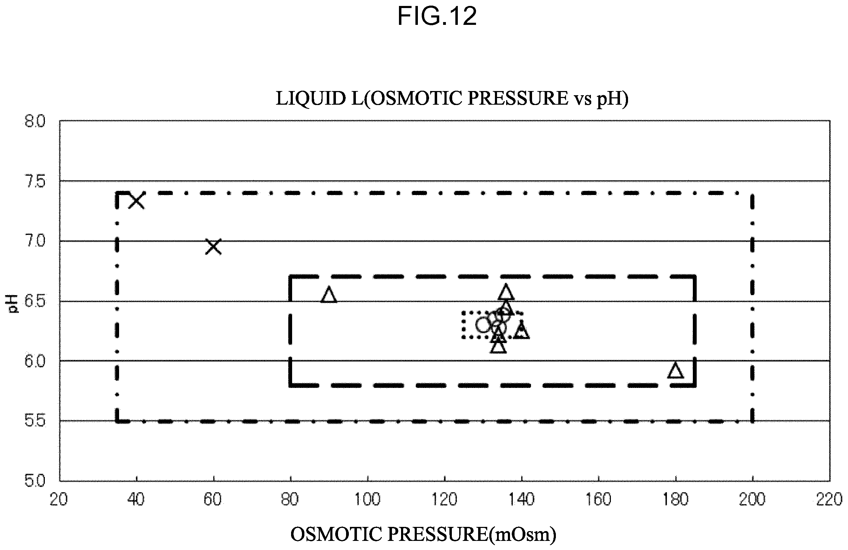

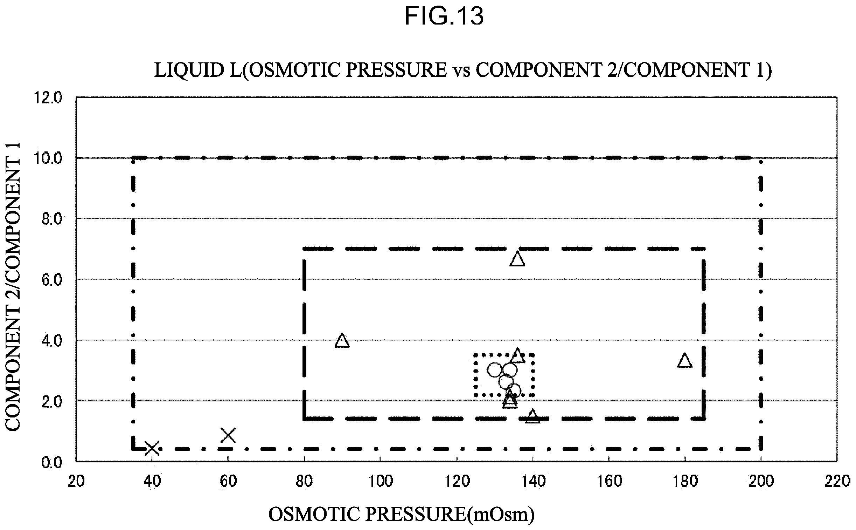

First, a range of the ratio of component 2/component 1, a preferred range of the osmotic pressure, and a preferred range of the pH of the eluent L were determined by mixing the component 1 and the component 2 at various content rates. The results are as presented in Table 6, Fig. 12, and Fig. 13. Fig. 12 illustrates the relationship between the osmotic pressure and the pH in the eluent L, and Fig. 13 illustrates the relationship between the osmotic pressure and the ratio of component 2/component 1 in the eluent L. Incidentally, the liquid L in Table 6 is an eluent having the same composition as the eluent L prepared in Example 1.

[Table 6]

| | Examina-tion 1 | Examina-tion 2 | Examina-tion 3 | Examina-tion 4 | Examina-tion 5 | Examina-tion 6 | Liquid L | Examina-tion 7 | Examina-tion 8 | Examina-tion 9 | Examina-tion 10 | Examina-tion 11 | Examina-tion 12 |
|---|---|---|---|---|---|---|---|---|---|---|---|---|---|
| Osmotic pressure | 40 | 60 | 136 | 90 | 136 | 135 | 133 | 130 | 134 | 140 | 134 | 134 | 180 |
| pH | 7.33 | 6.95 | 6.58 | 6.55 | 645 | 638 | 635 | 6.30 | 6.28 | 6.25 | 6.22 | 6.13 | 5.92 |
| Component (% by mass) | 0.097 | 0.161 | 0.360 | 0.173 | 0.300 | 0.270 | 0.248 | 0.216 | 0.225 | 0.218 | 0.200 | 0.180 | 0.165 |
| Compent 2 (% by mass) | 0.041 | 0.138 | 0.540 | 0.369 | 0.600 | 0.630 | 0.651 | 0.643 | 0.675 | 0.727 | 0.700 | 0.720 | 1.102 |
| Component 2/component 1 | 0.43 | 0.85 | 1.50 | 2.14 | 200 | 2.33 | 2.63 | 3.01 | 3.00 | 3.34 | 3.50 | 4.00 | 6.68 |
| Judgment | C | C | B | B | B | A | A | A | A | B | B | B | B |

The criteria of the judgment in Table 6, Fig. 12, and Fig. 13 are as follows.

A (○ in Fig. 12 and Fig. 13) ... HbA0, HbE, and HbD can be suitably separated.
B (△ in Fig. 12 and Fig. 13)... HbA0, HbE, and HbD can be separated but the peaks are too close to one another or it takes a long time to separate them.
C (✕ in Fig. 12 and Fig. 13) ... Two kinds among HbA0, HbE, and HbD can be separated.

In Fig. 12, the widest frame represented by an alternate long and short dash line indicates preferred ranges of the osmotic pressure and the pH of the eluent L, the frame represented by a broken line indicates more preferred ranges of the osmotic pressure and the pH of the eluent L, and the narrowest frame represented by a dotted line indicates still more preferred ranges of the osmotic pressure and the pH of the eluent L.

In Fig. 13, the widest frame represented by an alternate long and short dash line indicates a range of the ratio of component 2/component 1 and a preferred range of the osmotic pressure of the eluent L, the frame represented by a broken line indicates a preferred range of the ratio of component 2/component 1 and a more preferred range of the osmotic pressure of the eluent L, and the narrowest frame represented by a dotted line indicates a more preferred range of the ratio of component 2/component 1 and a still more preferred range of the osmotic pressure of the eluent L.

[Experiment 2]

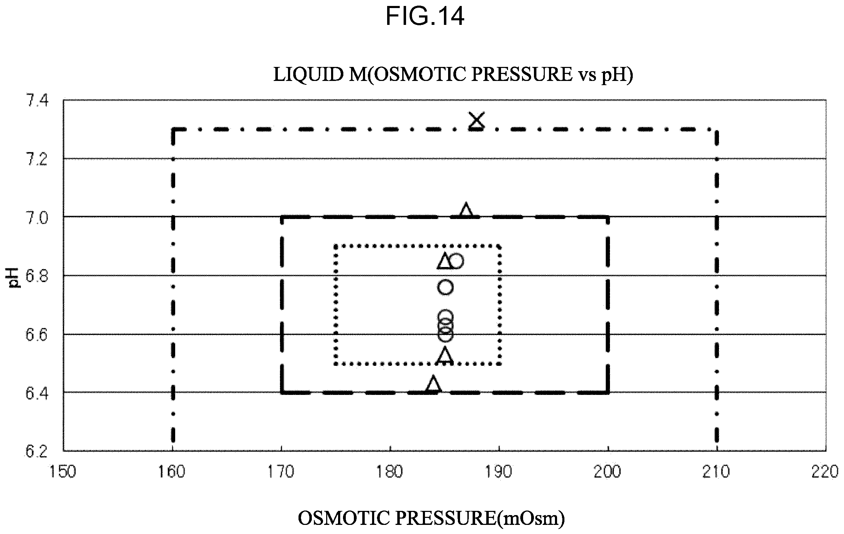

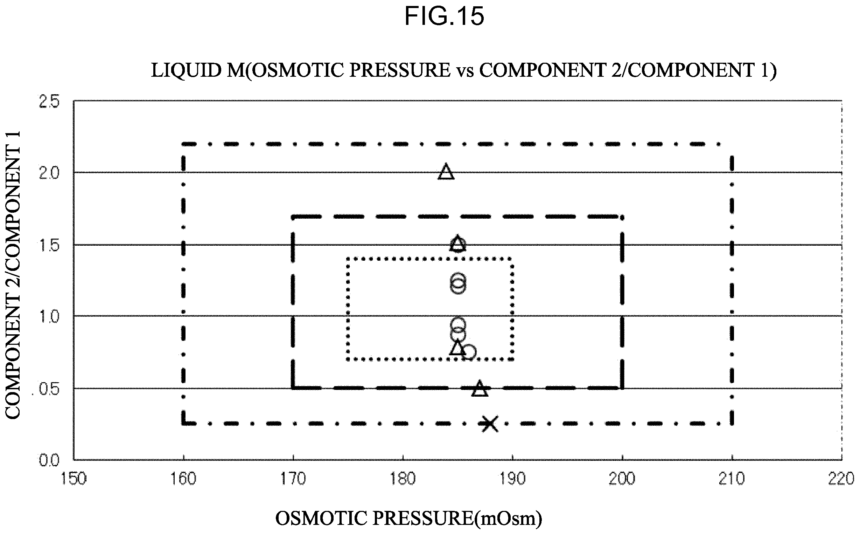

Next, a range of the ratio of component 2/component 1, a preferred range of the osmotic pressure, and a preferred range of the pH of the eluent M were determined by mixing the component 1 and the component 2 at various content rates. The results are as presented in Table 7, Fig. 14, and Fig. 15. Fig. 14 illustrates the relationship between the osmotic pressure and the pH in the eluent M, and Fig. 15 illustrates the relationship between the osmotic pressure and the ratio of component 2/component 1 in the eluent M. Incidentally, the liquid M in Table 7 is an eluent having the same composition as the eluent M prepared in Example 1.

[Table 7]

| | Examination 1 | Examination 2 | Examination 3 | Exammation 4 | Examination 5 | Liquid M | Examination 6 | Examination 7 | Examination 8 | Examination 9 | Examination 10 |
|---|---|---|---|---|---|---|---|---|---|---|---|
| Osmotic pressure | 188 | 187 | 185 | 186 | 185 | 185 | 185 | 185 | 185 | 185 | 184 |
| pH | 7.33 | 7.02 | 6.85 | 6.85 | 6.76 | 6.76 | 6.66 | 6.63 | 6.60 | 6.53 | 6.43 |
| Component 1 (% by mass) | 1.040 | 0.865 | 0.731 | 0.740 | 0.675 | 0.691 | 0.524 | 0.586 | 0.581 | 0.516 | 0.431 |
| Component 2 (% by mass) | 0.256 | 0.431 | 0.576 | 0.556 | 0.633 | 0.605 | 0.784 | 0710 | 0.727 | 0.780 | 0.865 |
| Componnent 2/component 1 | 0.25 | 0.50 | 0.79 | 0.75 | 0.94 | 0.88 | 1.25 | 1.21 | 1.50 | 1.51 | 2.01 |
| Judgment | C | B | B | A | A | A | A | A | A | B | B |

The criteria of the judgment in Table 7, Fig. 14, and Fig. 15 are as follows.

A (○ in Fig. 14 and Fig. 15)... HbC and HbCS can be suitably separated.
B (△ in Fig. 14 and Fig. 15)... HbC and HbCS can be separated but the peaks are too close to each other or it takes a long time to separate them.
C (✕ in Fig. 14 and Fig. 15)... HbC and HbCS cannot be separated.

In Fig. 14, the widest frame represented by an alternate long and short dash line indicates preferred ranges of the osmotic pressure and the pH of the eluent M, the frame represented by a broken line indicates more preferred ranges of the osmotic pressure and the pH of the eluent M, and the narrowest frame represented by a dotted line indicates still more preferred ranges of the osmotic pressure and the pH of the eluent M.

In Fig. 15, the widest frame represented by an alternate long and short dash line indicates a range of the ratio of component 2/component 1 and a preferred range of the osmotic pressure of the eluent M, the frame represented by a broken line indicates a preferred range of the ratio of component 2/component 1 and a more preferred range of the osmotic pressure of the eluent M, and the narrowest frame represented by a dotted line indicates a more preferred range of the ratio of component 2/component 1 and a still more preferred range of the osmotic pressure of the eluent M.

[Experiment 3]

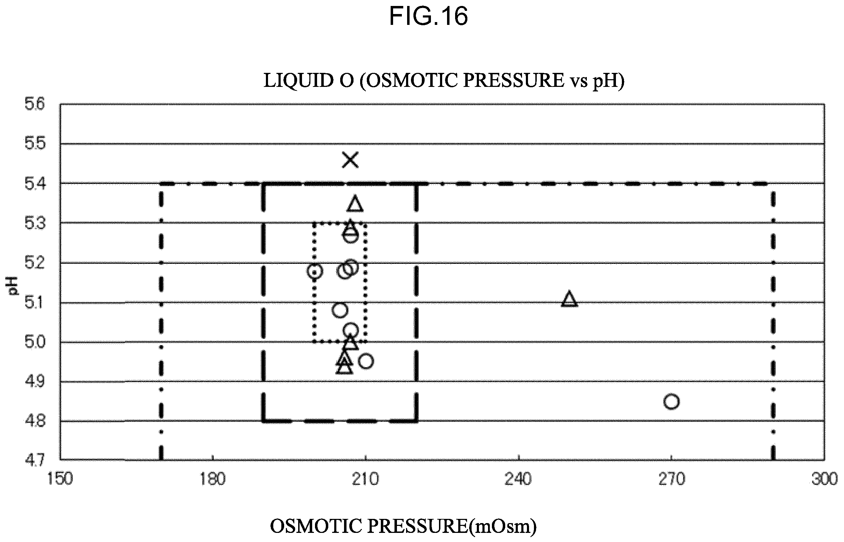

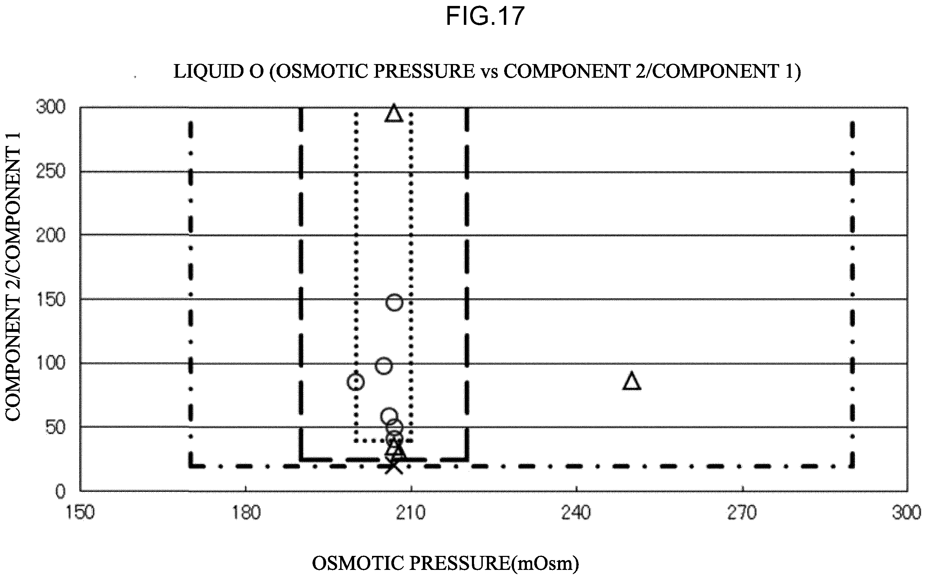

Next, a range of the ratio of component 2/component 1, a preferred range of the osmotic pressure, and a preferred range of the pH of the eluent O were determined by mixing the component 1 and the component 2 at various content rates. The results are as presented in Table 8, Fig. 16, and Fig. 17. Fig. 16 illustrates the relationship between the osmotic pressure and the pH in the eluent O, and Fig. 17 illustrates the relationship between the osmotic pressure and the ratio of component 2/component 1 in the eluent O. Incidentally, the liquid O in Table 8 is an eluent having the same composition as the eluent O prepared in Example 1.

[Table 8]

| | Examination 1 | Examination 2 | Examination 3 | Examination 4 | Examination 5 | Examination 6 | Liquid O | Examination 7 |
|---|---|---|---|---|---|---|---|---|
| Osmotic pressure | 207 | 208 | 207 | 207 | 207 | 200 | 206 | 250 |
| pH | 5.46 | 5.35 | 5.29 | 5.27 | 5.19 | 5.18 | 5.18 | 5.11 |
| Component 1 (% by mass) | 0.070 | 0.048 | 0.041 | 0.035 | 0.029 | 0.016 | 0.025 | 0.021 |
| Component 2 (% by mass) | 1.415 | 1.437 | 1.444 | 1.450 | 1.456 | 1.412 | 1.460 | 1.811 |
| Component 2/component 1 | 20.21 | 29.94 | 35.22 | 41.43 | 50.21 | 85.94 | 58.40 | 86.47 |
| Judgment | C | B | B | A | A | A | A | B |

| | Examination 8 | Examination 9 | Examination 10 | Examination 11 | Examination 12 | Examination 13 | Examination 14 |
|---|---|---|---|---|---|---|---|
| Osmotic pressure | 205 | 207 | 207 | 206 | 210 | 206 | 270 |
| pH | 5.08 | 5.03 | 5.00 | 4.96 | 4.95 | 4.94 | 4.85 |
| Component 1 (% by mass) | 0.015 | 0.010 | 0.005 | 0.003 | 0.002 | 0.000 | 0.002 |
| Component 2 (% by mass) | 1.470 | 1.475 | 1.480 | 1.483 | 1.508 | 1.485 | 1.991 |
| Component 2/component 1 | 98.00 | 147.50 | 296.00 | 593.00 | 890.91 | ∞ | 925.00 |
| Judgment | A | A | B | B | A | B | A |

The criteria of the judgment in Table 8, Fig. 16 and Fig. 17 are as follows.

A (○ in Fig. 16 and Fig. 17)... HbBart's and HbF can be suitably separated.
B (△ in Fig. 16 and Fig. 17)... HbBart's and HbF can be separated but the peaks are too close to each other or it takes a long time to separate them.
C (✕ in Fig. 16 and Fig. 17)... HbBart's and HbF cannot be separated.

In Fig. 16, the widest frame represented by an alternate long and short dash line indicates preferred ranges of the osmotic pressure and the pH of the eluent O, the frame represented by a broken line indicates more preferred ranges of the osmotic pressure and the pH of the eluent O, and the narrowest frame represented by a dotted line indicates still more preferred ranges of the osmotic pressure and the pH of the eluent O.

In Fig. 17, the widest frame represented by an alternate long and short dash line indicates a range of the ratio of component 2/component 1 and a preferred range of the osmotic pressure of the eluent O, the frame represented by a broken line indicates a preferred range of the ratio of component 2/component 1 and a more preferred range of the osmotic pressure of the eluent O, and the narrowest frame represented by a dotted line indicates a more preferred range of the ratio of component 2/component 1 and a still more preferred range of the osmotic pressure of the eluent O.

[Experiment 4]

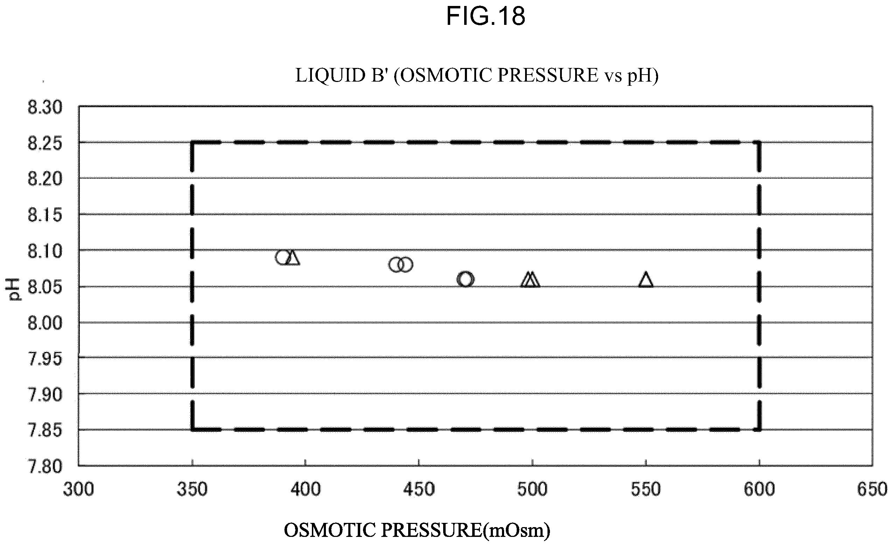

Next, the ranges of osmotic pressure and pH of the eluent B were determined. The results are as presented in Table 9 and Fig. 18. Fig. 18 illustrates the relationship between the osmotic pressure and the pH in the eluent B. Incidentally, the liquid B in Table 9 is an eluent having the same composition as the eluent B prepared in Example 1.

[Table 9]

| | Examination 1 | Examination 2 | Examination 3 | Examination 4 | Liquid B | Examination 5 | Examination 6 | Examination 7 | Examination 8 | Examination 9 |
|---|---|---|---|---|---|---|---|---|---|---|
| Osmotic pressure | 550 | 550 | 500 | 498 | 471 | 470 | 444 | 440 | 394 | 390 |
| pH | 8.06 | 8.05 | 8.06 | 8.06 | 8.06 | 8.06 | 8.08 | 8.08 | 8.09 | 8.09 |
| Judgment | B | B | B | B | A | A | A | A | B | A |

The criteria of the judgment in Table 9 and Fig. 18 are as follows.

A (○ in Fig. 18)... HbS, HbC, and HbCS can be suitably separated when the eluent B is supplied by mixing with the eluent A.

B (Δ in Fig. 18)... At least two kinds among HbS, HbC, and HbCS can be separated when the eluent B is supplied by mixing with the eluent A.

In Fig. 18, the ranges of the osmotic pressure and the pH of the eluent B are illustrated.

Incidentally, the same effect as the effect of Examples described above can be obtained using a column that is a different type from the column used in Examples described above as well.

In the liquid chromatographic system described above, the expression "can be measured" means that it can be eluted and is possibly separated from other Hb, and according to Examples, it is possible to measure predetermined hemoglobin using the eluent described above.

**Claims**

1. A measurement method of measuring at least one kind of hemoglobin selected from abnormal hemoglobin or hemoglobin that is a marker for thalassemia in accordance with any one of the following (A) to (D):

   (A) in high-performance liquid chromatography, an eluent L that contains a phosphoric acid monohydrogen dialkali metal salt (component 1) and a phosphoric acid dihydrogen monoalkali metal salt (component 2) as components and that has a content of component 1 of from 0.08% by mass to 0.50% by mass with respect to a total mass of the eluent L, a content of component 2 of from 0.04% by mass to 1.2% by mass with respect to a total mass of the eluent L, and a ratio of component 2/component 1 of from 0.4 to 10 is used;
   (B) in high-performance liquid chromatography, an eluent O that contains a phosphoric acid monohydrogen dialkali metal salt (component 1) and a phosphoric acid dihydrogen monoalkali metal salt (component 2) as components and that has a content of component 1 of less than 0.07% by mass with respect to a total mass of the eluent O, a content of component 2 of from more than 1.4% by mass to 2.0% by mass with respect to a total mass of the eluent O, and a ratio of component 2/component 1 of more than 20 is used;
   (C) in high-performance liquid chromatography, an eluent M that contains a phosphoric acid monohydrogen dialkali metal salt (component 1) and a phosphoric acid dihydrogen monoalkali metal salt (component 2) as components and that has a content of component 1 of from 0.40% by mass to less than 1.04% by mass with respect to a total mass of the eluent M, a content of component 2 of from more than 0.26% by mass to 0.88% by mass with respect to a total mass of the eluent M, and a ratio of component 2/component 1 of from more than 0.25 to 2.2 is used; and
   (D) in high-performance liquid chromatography, at least two eluents selected from the group consisting of an eluent L that contains a phosphoric acid monohydrogen dialkali metal salt (component 1) and a phosphoric acid dihydrogen monoalkali metal salt (component 2) as components and that has a content of component 1 of from 0.08% by mass to 0.50% by mass with respect to a total mass of the eluent L, a content of component 2 of from 0.04% by mass to 1.2% by mass with respect to a total mass of the eluent L, and a ratio of component 2/component 1 of from 0.4 to 10, an eluent O that has a content of component 1 of less than 0.07% by mass with respect to a total mass of the eluent O, a content of component 2 of from more than 1.4% by mass to 2.0% by mass with respect to a total mass of the eluent O, and a ratio of component 2/component 1 of more than 20, and an eluent M that has a content of component 1 of from 0.40% by mass to less than 1.04% by mass with respect to a total mass of the eluent M, a content of component 2 of from more than 0.26% by mass to 0.88% by mass with respect to a total mass of the eluent M, and a ratio of component 2/component 1 of from more than 0.25 to 2.2 are used in combination.

2. The measurement method according to claim 1, wherein the at least two eluents are used in accordance with (D) above and any one of the following (I) to (III):

   (I) the at least two eluents selected from the group consisting of the eluent L, the eluent O, and the eluent M are used successively without mixing;
   (II) at least one mixed liquid prepared by mixing the at least two eluents selected from the group consisting of the eluent L, the eluent O, and the eluent M is used; or
   (III) at least one eluent selected from the group consisting of the eluent L, the eluent O, and the eluent M, and at least one mixed liquid prepared by mixing the at least two eluents selected from the group consisting of the eluent L, the eluent O, and the eluent M are used successively.

3. The measurement method according to claim 1 or claim 2, wherein mixed liquids are used in accordance with (D) above and the following (a) or (b):

    (a) at least one mixed liquid prepared by mixing at least one of the eluent O or the eluent M with the eluent L is used; and
    (b) the eluent L, a mixed liquid prepared by mixing the eluent L and the eluent O, and a mixed liquid prepared by mixing the eluent M and the eluent L are used.

4. The measurement method according to claim 3, wherein at least one hemoglobin variant of HbBart's, HbH, HbF, HbA1c, HbA0, HbA2, HbE, HbD, HbS, HbC, or HbConstantSpring is measured by (b) above.

5. The measurement method according to any one of claims 1 to 4, wherein at least one of the following (1) to (5) is satisfied:

    (1) an osmotic pressure of the eluent L is from 35 mOsm to 200 mOsm;
    (2) an osmotic pressure of the eluent O is from 170 mOsm to 290 mOsm;
    (3) an osmotic pressure of the eluent M is from 160 mOsm to 210 mOsm;
    (4) a mixed liquid prepared by mixing the eluent L and the eluent O is used, and a mixing ratio of the eluents is eluent L : eluent O is from 1:2 to 1:4; and
    (5) a mixed liquid prepared by mixing the eluent M and the eluent L is used, and a mixing ratio of the eluents is eluent M : eluent L is from 1:2 to 1:4.

6. The measurement method according to any one of claims 1 to 5, wherein at least one of the following (6) to (8) is satisfied:

    (6) an eluent A that has a pH of from 5.30 to 5.40 and an osmotic pressure of from 204 mOsm to 210 mOsm is further used;
    (7) an eluent B that has a pH of from 7.85 to 8.25 and an osmotic pressure of from 350 mOsm to 600 mOsm is further used; and
    (8) a mixed liquid prepared by mixing an eluent B that has a pH of from 7.85 to 8.25 and an osmotic pressure of from 350 mOsm to 600 mOsm and an eluent A that has a pH of from 5.30 to 5.40 and an osmotic pressure of from 204 mOsm to 210 mOsm at a mixing ratio of eluent B: eluent A of from 1:2 to 1:4 is further used.

7. The measurement method according to claim 6, wherein at least one hemoglobin variant of HbBart's, HbH, HbF, HbA1c, HbA0, HbA2, HbE, HbD, HbS, HbC, or HbConstantSpring is measured by (8) above.

8. A measurement apparatus for measuring a hemoglobin variant by high-performance liquid chromatography, the measurement apparatus comprising:

    a supply pump to supply, singly or in combination, one or more eluents selected from the group consisting of an eluent L that contains a phosphoric acid monohydrogen dialkali metal salt (component 1) and a phosphoric acid dihydrogen monoalkali metal salt (component 2) as components and that has a content of component 1 of from 0.08% by mass to 0.50% by mass with respect to a total mass of the eluent L, a content of component 2 of from 0.04% by mass to 1.2% by mass with respect to a total mass of the eluent L, and a ratio of component 2/component 1 of from 0.4 to 10, an eluent O that has a content of component 1 of less than 0.07% by mass with respect to a total mass of the eluent O, a content of component 2 of from more than 1.4% by mass to 2.0% by mass with respect to a total mass of the eluent O, and a ratio of component 2/component 1 of more than 20, and an eluent M that has a content of component 1 of from 0.40% by mass to less than 1.04% by mass with respect to a total mass of the eluent M, a content of component 2 of from more than 0.26% by mass to 0.88% by mass with respect to a total mass of the eluent M, and a ratio of component 2/component 1 of from more than 0.25 to 2.2;
    a separation column which is connected to the supply pump and to which the eluent is supplied; and
    a plurality of eluent tanks storing each of eluent L, eluent O and eluent M.

9. The measurement apparatus according to claim 8, wherein the supply pump supplies eluents in accordance with any one of the following (I) to (III):

(I) at least one eluent selected from the group consisting of the eluent L, the eluent O, and the eluent M is supplied, or at least two eluents selected from the group consisting of the eluent L, the eluent O, and the eluent M are supplied successively without mixing;

(II) at least one mixed liquid prepared by mixing the at least two eluents selected from the group consisting of the eluent L, the eluent O, and the eluent M, is supplied; and

(III) at least one eluent selected from the group consisting of the eluent L, the eluent O, and the eluent M, and at least one mixed liquid prepared by mixing the at least two eluents selected from the group consisting of the eluent L, the eluent O, and the eluent M, are supplied successively.

10. The measurement apparatus according to claim 8 or claim 9, wherein, as the eluent, at least one of an eluent A that has a pH of from 5.30 to 5.40 and an osmotic pressure of from 204 mOsm to 210 mOsm or an eluent B that has a pH of from 7.85 to 8.25 and an osmotic pressure of from 350 mOsm to 600 mOsm, is further supplied, or a mixed liquid prepared by mixing the eluent A and the eluent B is further supplied.

11. The measurement apparatus according to any one of claims 8 to 10, wherein the apparatus has a single supply pump.

12. An eluent being any one of the following (1) to (3);

(1) An eluent L suitable for measuring at least one kind of hemoglobin selected from abnormal hemoglobin or hemoglobin that is a marker for thalassemia by high-performance liquid chromatography, the eluent comprising:

a phosphoric acid monohydrogen dialkali metal salt (component 1); and
a phosphoric acid dihydrogen monoalkali metal salt (component 2), wherein
a content of component 1 is from 0.08% by mass to 0.50% by mass with respect to a total mass of the eluent L,
a content of component 2 is from 0.04% by mass to 1.2% by mass with respect to a total mass of the eluent L, and
a ratio of component 2/component 1 is from 0.4 to 10;

(2) An eluent O suitable for measuring at least one kind of hemoglobin selected from abnormal hemoglobin or hemoglobin that is a marker for thalassemia by high-performance liquid chromatography, the eluent comprising:

a phosphoric acid monohydrogen dialkali metal salt (component 1); and
a phosphoric acid dihydrogen monoalkali metal salt (component 2), wherein
a content of component 1 is less than 0.07% by mass with respect to a total mass of the eluent O,
a content of component 2 is from more than 1.4% by mass to 2.0% by mass with respect to a total mass of the eluent O, and
a ratio of component 2/component 1 is more than 20; and

(3) An eluent M suitable for measuring at least one kind of hemoglobin selected from abnormal hemoglobin or hemoglobin that is a marker for thalassemia by high-performance liquid chromatography, the eluent comprising:

a phosphoric acid monohydrogen dialkali metal salt (component 1); and
a phosphoric acid dihydrogen monoalkali metal salt (component 2), wherein
a content of component 1 is from 0.40% by mass to less than 1.04% by mass with respect to a total mass of the eluent M,
a content of component 2 is from more than 0.26% by mass to 0.88% by mass with respect to a total mass of the eluent M, and
a ratio of component 2/component 1 is from more than 0.25 to 2.2.

13. A combination of eluents suitable for measuring at least one kind of hemoglobin, selected from abnormal hemoglobin or hemoglobin that is a marker for thalassemia, by high-performance liquid chromatography, the combination of eluents comprising,
at least two eluents selected from the group consisting of an eluent L that contains a phosphoric acid monohydrogen dialkali metal salt (component 1) and a phosphoric acid dihydrogen monoalkali metal salt (component 2) as components and that has a content of component 1 of from 0.08% by mass to 0.50% by mass with respect to a total mass of the eluent L, a content of component 2 of from 0.04% by mass to 1.2% by mass with respect to a total mass of the eluent L, and a ratio of component 2/component 1 of from 0.4 to 10; an eluent O that has a content of component 1 of less than 0.07% by mass with respect to a total mass of the eluent O, a content of component 2 of from more than 1.4% by mass to 2.0% by mass with respect to a total mass of the eluent O, and a ratio of component 2/component 1 of more than 20; and an eluent M that has a content of component 1 of from 0.40% by mass to less than 1.04% by mass with respect to a total mass of the eluent M, a content of component 2 of from more than 0.26% by mass to 0.88% by mass with respect to a total mass of the eluent M, and a ratio of component 2/component 1 of from more than 0.25 to 2.2.

**Patentansprüche**

1. Ein Messverfahren zur Messung von wenigstens einer Hämoglobin-Art ausgewählt aus anormalem Hämoglobin oder Hämoglobin, das ein Marker für Thalassämie ist, in Übereinstimmung mit einem von den Folgenden (A) bis (D):

(A) in Hochleistungs-Flüssigkeitschromatografie ein Eluent L, der ein Phosphorsäuremonohydrogendialkalimetallsalz (Komponente 1) und ein Phosphorsäuredihydrogenmonoalkalimetallsalz (Komponente 2) als Komponenten enthält und der einen Gehalt von Komponente 1 von 0,08 Masse-% bis 0,50 Masse-% bezogen auf eine Gesamtmasse von dem Eluenten L, einen Gehalt von Komponente 2 von 0,04 Masse-% bis 1,2 Masse-% bezogen auf eine Gesamtmasse von dem Eluenten L und ein Verhältnis von Komponente 2/Komponente 1 von 0,4 bis 10 hat, verwendet wird;

(B) in Hochleistungs-Flüssigkeitschromatografie ein Eluent O, der ein Phosphorsäuremonohydrogendialkalimetallsalz (Komponente 1) und ein Phosphorsäuredihydrogenmonoalkalimetallsalz (Komponente 2) als Komponenten enthält und der einen Gehalt von Komponente 1 von weniger als 0,07 Masse-% bezogen auf eine Gesamtmasse von dem Eluenten O, einen Gehalt von Komponente 2 von mehr als 1,4 Masse-% bis 2,0 Masse-% bezogen auf eine Gesamtmasse von dem Eluenten O und ein Verhältnis von Komponente 2/Komponente 1 von mehr als 20 hat, verwendet wird;

(C) in Hochleistungs-Flüssigkeitschromatografie ein Eluent M, der ein Phosphorsäuremonohydrogendialkalimetallsalz (Komponente 1) und ein Phosphorsäuredihydrogenmonoalkalimetallsalz (Komponente 2) als Komponenten enthält und der einen Gehalt von Komponente 1 von 0,40 Masse-% bis weniger als 1,04 Masse-% bezogen auf eine Gesamtmasse von dem Eluenten M, einen Gehalt von Komponente 2 von mehr als 0,26 Masse-% bis 0,88 Masse-% bezogen auf eine Gesamtmasse von dem Eluenten M und ein Verhältnis von Komponente 2/Komponente 1 von mehr als 0,25 bis 2,2 hat, verwendet wird; und

(D) in Hochleistungs-Flüssigkeitschromatografie wenigstens zwei Eluenten ausgewählt aus der Gruppe bestehend aus einem Eluenten L, der ein Phosphorsäuremonohydrogendialkalimetallsalz (Komponente 1) und ein Phosphorsäuredihydrogenmonoalkalimetallsalz (Komponente 2) als Komponenten enthält und der einen Gehalt von Komponente 1 von 0,08 Masse-% bis 0,50 Masse-% bezogen auf eine Gesamtmasse von dem Eluenten L, einen Gehalt von Komponente 2 von 0,04 Masse-% bis 1,2 Masse-% bezogen auf eine Gesamtmasse von dem Eluenten L und ein Verhältnis von Komponente 2/Komponente 1 von 0,4 bis 10 hat, einem Eluenten O, der einen Gehalt von Komponente 1 von weniger als 0,07 Masse-% bezogen auf eine Gesamtmasse von dem Eluenten O, einen Gehalt von Komponente 2 von mehr als 1,4 Masse-% bis 2,0 Masse-% bezogen auf eine Gesamtmasse von dem Eluenten O und ein Verhältnis von Komponente 2/Komponente 1 von mehr als 20 hat, und einem Eluenten M, der einen Gehalt von Komponente 1 von 0,40 Masse-% bis weniger als 1,04 Masse-% bezogen auf eine Gesamtmasse von dem Eluenten M, einen Gehalt von Komponente 2 von mehr als 0,26 Masse-% bis 0,88 Masse-% bezogen auf eine Gesamtmasse von dem Eluenten M und ein Verhältnis von Komponente 2/Komponente 1 von mehr als 0,25 bis 2,2 hat, werden in Kombination verwendet.

2. Das Messverfahren nach Anspruch 1, wobei die wenigstens zwei Eluenten in Übereinstimmung mit vorstehendem (D) und einem von den Folgenden (I) bis (III) verwendet werden:

(I) die wenigstens zwei Eluenten ausgewählt aus der Gruppe bestehend aus dem Eluenten L, dem Eluenten O und dem Eluenten M werden nacheinander ohne Mischen verwendet;

(II) wenigstens eine gemischte Flüssigkeit, die durch Mischen der wenigstens zwei Eluenten ausgewählt aus der Gruppe bestehend aus dem Eluenten L, dem Eluenten O und dem Eluenten M hergestellt ist, wird verwendet; oder

(III) wenigstens ein Eluent ausgewählt aus der Gruppe bestehend aus dem Eluenten L, dem Eluenten O und dem Eluenten M und wenigstens eine gemischte Flüssigkeit, die durch Mischen der wenigstens zwei Eluenten ausgewählt aus der Gruppe bestehend aus dem Eluenten L, dem Eluenten O und dem Eluenten M hergestellt ist, werden nacheinander verwendet.

3. Das Messverfahren nach Anspruch 1 oder Anspruch 2, wobei gemischte Flüssigkeiten in Übereinstimmung mit vorstehendem (D) und dem Folgenden (a) oder (b) verwendet werden:

(a) wenigstens eine gemischte Flüssigkeit, die durch Mischen wenigstens eines von dem Eluenten O oder dem Eluenten M mit dem Eluenten L hergestellt ist, wird verwendet; und

(b) der Eluent L, eine gemischte Flüssigkeit, die durch Mischen des Eluenten L und des Eluenten O hergestellt ist, und eine gemischte Flüssigkeit, die durch Mischen des Eluenten M und des Eluenten L hergestellt ist, werden verwendet.

4. Das Messverfahren nach Anspruch 3, wobei wenigstens eine Hämoglobin-Variante von HbBart's, HbH, HbF, HbA1c, HbA0, HbA2, HbE, HbD, HbS, HbC oder HbConstantSpring durch vorstehendes (b) gemessen wird.

5. Das Messverfahren nach einem der Ansprüche 1 bis 4, wobei wenigstens eines von den Folgenden (1) bis (5) erfüllt ist:

(1) ein osmotischer Druck von dem Eluenten L ist von 35 mOsm bis 200 mOsm;
(2) ein osmotischer Druck von dem Eluenten O ist von 170 mOsm bis 290 mOsm;
(3) ein osmotischer Druck von dem Eluenten M ist von 160 mOsm bis 210 mOsm;
(4) eine gemischte Flüssigkeit, die durch Mischen des Eluenten L und des Eluenten O hergestellt ist, wird verwendet und ein Mischverhältnis von den Eluenten ist Eluent L : Eluent O ist von 1:2 bis 1:4; und
(5) eine gemischte Flüssigkeit, die durch Mischen des Eluenten M und des Eluenten L hergestellt ist, wird verwendet und ein Mischverhältnis von den Eluenten ist Eluent M : Eluent L ist von 1:2 bis 1:4.

6. Das Messverfahren nach einem der Ansprüche 1 bis 5, wobei wenigstens eines von den Folgenden (6) bis (8) erfüllt ist:

(6) ein Eluent A, der einen pH von 5,30 bis 5,40 und einen osmotischen Druck von 204 mOsm bis 210 mOsm hat, wird weiter verwendet;
(7) ein Eluent B, der einen pH von 7,85 bis 8,25 und einen osmotischen Druck von 350 mOsm bis 600 mOsm hat, wird weiter verwendet; und
(8) eine gemischte Flüssigkeit, die durch Mischen eines Eluenten B, der einen pH von 7,85 bis 8,25 und einen osmotischen Druck von 350 mOsm bis 600 mOsm hat, und eines Eluenten A, der einen pH von 5,30 bis 5,40 und einen osmotischen Druck von 204 mOsm bis 210 mOsm hat, mit einem Mischverhältnis von Eluent B : Eluent A von 1:2 bis 1:4 hergestellt ist, wird weiter verwendet.

7. Das Messverfahren nach Anspruch 6, wobei wenigstens eine Hämoglobin-Variante von HbBart's, HbH, HbF, HbA1c, HbA0, HbA2, HbE, HbD, HbS, HbC oder HbConstantSpring durch vorstehendes (8) gemessen wird.

8. Eine Messvorrichtung zur Messung einer Hämoglobin-Variante durch Hochleistungs-Flüssigkeitschromatografie, die Messvorrichtung umfassend:

eine Versorgungspumpe zum Liefern, einzeln oder in Kombination, eines oder mehrerer Eluenten ausgewählt aus der Gruppe bestehend aus einem Eluenten L, der ein Phosphorsäuremonohydrogendialkalimetallsalz (Komponente 1) und ein Phosphorsäuredihydrogenmonoalkalimetallsalz (Komponente 2) als Komponenten enthält und der einen Gehalt von Komponente 1 von 0,08 Masse-% bis 0,50 Masse-% bezogen auf eine Gesamtmasse von dem Eluenten L, einen Gehalt von Komponente 2 von 0,04 Masse-% bis 1,2 Masse-% bezogen auf eine Gesamtmasse von dem Eluenten L und ein Verhältnis von Komponente 2/Komponente 1 von 0,4 bis 10 hat, einem Eluenten O, der einen Gehalt von Komponente 1 von weniger als 0,07 Masse-% bezogen auf eine Gesamtmasse von dem Eluenten O, einen Gehalt von Komponente 2 von mehr als 1,4 Masse-% bis 2,0 Masse-% bezogen auf eine Gesamtmasse von dem Eluenten O und ein Verhältnis von Komponente 2/Komponente 1 von mehr als 20 hat, und einem Eluenten M, der einen Gehalt von Komponente 1 von 0,40 Masse-% bis weniger als 1,04 Masse-% bezogen auf eine Gesamtmasse von dem Eluenten M, einen Gehalt von Komponente 2 von mehr als 0,26 Masse-% bis 0,88 Masse-% bezogen auf eine Gesamtmasse von dem Eluenten M und ein Verhältnis von Komponente 2/Komponente 1 von mehr als 0,25 bis 2,2 hat;
eine Trennsäule, die mit der Versorgungspumpe verbunden ist und zu der der Eluent geliefert wird; und
eine Vielzahl von Eluententanks, die jeden von dem Eluenten L, dem Eluenten O und dem Eluenten M speichert.

9. Die Messvorrichtung nach Anspruch 8, wobei die Versorgungspumpe Eluenten in Übereinstimmung mit einem von den Folgenden (I) bis (III) liefert:

(I) wenigstens ein Eluent ausgewählt aus der Gruppe bestehend aus dem Eluenten L, dem Eluenten O und dem Eluenten M wird geliefert oder wenigstens zwei Eluenten ausgewählt aus der Gruppe bestehend aus dem Eluenten L, dem Eluenten O und dem Eluenten M sind nacheinander ohne Mischen geliefert;

(II) wenigstens eine gemischte Flüssigkeit, die durch Mischen der wenigstens zwei Eluenten ausgewählt aus der Gruppe bestehend aus dem Eluenten L, dem Eluenten O und dem Eluenten M hergestellt ist, ist geliefert; und

(III) wenigstens ein Eluent ausgewählt aus der Gruppe bestehend aus dem Eluenten L, dem Eluenten O und dem Eluenten M und wenigstens eine gemischte Flüssigkeit, die durch Mischen der wenigstens zwei Eluenten ausgewählt aus der Gruppe bestehend aus dem Eluenten L, dem Eluenten O und dem Eluenten M hergestellt ist, sind nacheinander geliefert.

10. Die Messvorrichtung nach Anspruch 8 oder Anspruch 9, wobei als Eluent wenigstens einer von einem Eluenten A, der einen pH von 5,30 bis 5,40 und einen osmotischen Druck von 204 mOsm bis 210 mOsm hat, oder einem Eluenten B, der einen pH von 7,85 bis 8,25 und einen osmotischen Druck von 350 mOsm bis 600 mOsm hat, weiter geliefert ist oder eine gemischte Flüssigkeit, die durch Mischen des Eluenten A und des Eluenten B hergestellt ist, weiter geliefert ist.

11. Die Messvorrichtung nach einem der Ansprüche 8 bis 10, wobei die Vorrichtung eine einzige Versorgungspumpe hat.

12. Ein Eluent, der einer von den Folgenden (1) bis (3) ist:

(1) ein Eluent L, der dazu geeignet ist, wenigstens eine Hämoglobin-Art ausgewählt aus einem anormalen Hämoglobin oder einem Hämoglobin, das ein Marker für Thalassämie ist, durch Hochleistungschromatografie zu messen, wobei der Eluent umfasst:

ein Phosphorsäuremonohydrogendialkalimetallsalz (Komponente 1); und
ein Phosphorsäuredihydrogenmonoalkalimetallsalz (Komponente 2), wobei ein Gehalt von Komponente 1 von 0,08 Masse-% bis 0,50 Masse-% bezogen auf eine Gesamtmasse von dem Eluenten L ist,
ein Gehalt von Komponente 2 von 0,04 Masse-% bis 1,2 Masse-% bezogen auf eine Gesamtmasse von dem Eluenten L ist; und
ein Verhältnis von Komponente 2/Komponente 1 von 0,4 bis 10 ist;

(2) ein Eluent O, der dazu geeignet ist, wenigstens eine Hämoglobin-Art ausgewählt aus einem anormalen Hämoglobin oder einem Hämoglobin, das ein Marker für Thalassämie ist, durch Hochleistungs-Flüssigkeitschromatografie zu messen, wobei der Eluent umfasst:

ein Phosphorsäuremonohydrogendialkalimetallsalz (Komponente 1); und
ein Phosphorsäuredihydrogenmonoalkalimetallsalz (Komponente 2), wobei ein Gehalt von Komponente 1 geringer als 0,07 Masse-% bezogen auf eine Gesamtmasse von dem Eluenten O ist,
ein Gehalt von Komponente 2 mehr als 1,4 Masse-% bis 2,0 Masse-% bezogen auf eine Gesamtmasse von dem Eluenten O ist, und
ein Verhältnis von Komponente 2/Komponente 1 mehr als 20 ist; und

(3) ein Eluent M, der dazu geeignet ist, wenigstens eine Hämoglobin-Art ausgewählt aus einem anormalen Hämoglobin oder einem Hämoglobin, das ein Marker für Thalassämie ist, durch Hochleistungs-Flüssigkeitschromatografie zu messen, wobei der Eluent umfasst:

ein Phosphorsäuremonohydrogendialkalimetallsalz (Komponente 1); und
ein Phosphorsäuredihydrogenmonoalkalimetallsalz (Komponente 2), wobei ein Gehalt von Komponente 1 von 0,40 Masse-% bis weniger als 1,04 Masse-% bezogen auf eine Gesamtmasse von dem Eluenten M ist,
ein Gehalt von Komponente 2 von mehr als 0,26 Masse-% bis 0,88 Masse-% bezogen auf eine Gesamtmasse von dem Eluenten M ist, und
ein Verhältnis von Komponente 2/Komponente 1 von mehr als 0,25 bis 2,2 ist.

13. Eine Kombination von Eluenten, die zur Messung wenigstens einer Hämoglobin-Art durch Hochleistungs-Flüssigkeitschromatografie geeignet und aus einem anormalen Hämoglobin oder einem Hämoglobin, das ein Marker für Thalassämie ist, ausgewählt sind, wobei die Kombination von Eluenten umfasst:

wenigstens zwei Eluenten ausgewählt aus der Gruppe bestehend aus einem Eluenten L, der ein Phosphorsäuremonohydrogendialkalimetallsalz (Komponente 1) und ein Phosphorsäuredihydrogenmonoalkalimetallsalz (Komponente 2) als Komponenten enthält und der einen Gehalt von Komponente 1 von 0,08 Masse-% bis 0,50 Masse-% bezogen auf eine Gesamtmasse von dem Eluenten L, einen Gehalt von Komponente 2 von 0,04 Masse-% bis 1,2 Masse-% bezogen auf eine Gesamtmasse von dem Eluenten L und ein Verhältnis von Komponente 2/Komponente 1 von 0,4 bis 10 hat, einem Eluenten O, der einen Gehalt von Komponente 1 von weniger als 0,07 Masse-% bezogen auf eine Gesamtmasse von dem Eluenten O, einen Gehalt von Komponente 2 von mehr als 1,4 Masse-% bis 2,0 Masse-% bezogen auf eine Gesamtmasse von dem Eluenten O und ein Verhältnis von Komponente 2/Komponente 1 von mehr als 20 hat und einem Eluenten M, der einen Gehalt von Komponente 1 von 0,4 Masse-% bis weniger als 1,04 Masse-% bezogen auf eine Gesamtmasse von dem Eluenten M, einen Gehalt von Komponente 2 von mehr als 0,26 Masse-% bis 0,88 Masse-% bezogen auf eine Gesamtmasse von dem Eluenten M und ein Verhältnis von Komponente 2/Komponente 1 von 0,25 bis 2,2 hat.

## Revendications

1. 1. Procédé de mesure pour la mesure d'au moins un type d'hémoglobine choisi parmi une hémoglobine anormale ou une hémoglobine qui est un marqueur de la thalassémie conformément à l'un quelconque des articles (A) à (D) suivants :

(A) en chromatographie en phase liquide de hautes performances, on utilise un éluant L qui contient un sel de métal dialcalin monohydrogéné d'acide phosphorique (composant 1) et un sel de métal monoalcalin dihydrogéné d'acide phosphorique (composant 2) comme composants et qui a une teneur en composant 1 de 0,08 % en masse à 0,50 % en masse par rapport à une masse totale de l'éluant L, une teneur en composant 2 de 0,04 % en masse à 1,2 % en masse par rapport à une masse totale de l'éluant L, et un rapport du composant 2 au composant 1 de 0,4 à 10 ;
(B) en chromatographie en phase liquide de hautes performances, on utilise un éluant O qui contient un sel de métal dialcalin monohydrogéné d'acide phosphorique (composant 1) et un sel de métal monoalcalin dihydrogéné d'acide phosphorique (composant 2) comme composants et qui a une teneur en composant 1 de moins de 0,07 % en masse par rapport à une masse totale de l'éluant O, une teneur en composant 2 de plus de 1,4 % en masse à 2,0 % en masse par rapport à une masse totale de l'éluant O, et un rapport du composant 2 au composant 1 de plus de 20 ;
(C) en chromatographie en phase liquide de hautes performances, un éluant M qui contient un sel de métal dialcalin monohydrogéné d'acide phosphorique (composant 1) et un sel de métal monoalcalin dihydrogéné d'acide phosphorique (composant 2) comme composants et qui a une teneur en composant 1 de 0,40 % en masse à moins de 1,04 % en masse par rapport à une masse totale de l'éluant M, une teneur en composant 2 de plus de 0,26 % en masse à 0,88 % en masse par rapport à une masse totale de l'éluant M, et un rapport du composant 2 au composant 1 de plus de 0,25 à 2,2 est utilisé ; et
(D) en chromatographie en phase liquide de hautes performances, au moins deux éluants choisis dans le groupe constitué d'un éluant L qui contient un sel de métal dialcalin monohydrogéné d'acide phosphorique (composant 1) et un sel de métal monoalcalin dihydrogéné d'acide phosphorique (composant 2) comme composants et qui a une teneur en composant 1 de 0,08 % en masse à 0,50 % en masse par rapport à une masse totale de l'éluant L, une teneur en composant 2 de 0,04 % en masse à 1,2 % en masse par rapport à une masse totale de l'éluant L, et un rapport du composant 2 au composant 1 de 0,4 à 10, un éluant O qui a une teneur en composant 1 de moins de 0,07 % en masse par rapport à une masse totale de l'éluant O, une teneur en composant 2 de plus de 1,4 % en masse à 2,0 % en masse par rapport à une masse totale de l'éluant O, et un rapport du composant 2 au composant 1 de plus de 20, et un éluant M qui a une teneur en composant 1 de 0,40 % en masse à moins de 1,04 % en masse par rapport à une masse totale de l'éluant M, une teneur en composant 2 de plus de 0,26 % en masse à 0,88 % en masse par rapport à une masse totale de l'éluant M, et un rapport du composant 2 au composant 1 de plus de 0,25 à 2,2 sont utilisés en combinaison.

2. Procédé de mesure selon la revendication 1, dans lequel les au moins deux éluants sont utilisés conformément à (D) ci-dessus et selon l'un quelconque des articles (I) à (III) suivants :

(I) les au moins deux éluants choisis dans le groupe constitué de l'éluant L, de l'éluant O et de l'éluant M sont utilisés successivement sans mélange ;
(II) au moins un liquide mixte préparé en mélangeant les au moins deux éluants choisis dans le groupe constitué de l'éluant L, de l'éluant O et de l'éluant M est utilisé ; ou
(III) au moins un éluant choisi dans le groupe constitué de l'éluant L, de l'éluant O et de l'éluant M, et au moins un liquide mixte préparé en mélangeant les au moins deux éluants choisis dans le groupe constitué de l'éluant L, l'éluant O et l'éluant M sont utilisés successivement.

3. Procédé de mesure selon la revendication 1 ou la revendication 2, dans lequel des liquides mixtes sont utilisés selon (D) ci-dessus et les articles (a) ou (b) suivants :

(a) au moins un liquide mixte préparé en mélangeant au moins l'un de l'éluant O ou de l'éluant M avec l'éluant L est utilisé ; et
(b) l'éluant L, un liquide mixte préparé en mélangeant l'éluant L et l'éluant O et un liquide mixte préparé en mélangeant l'éluant M et l'éluant L sont utilisés.

4. Procédé de mesure selon la revendication 3, dans lequel au moins un variant d'hémoglobine de HbBart's, HbH, HbF, HbA1c, HbA0, HbA2, HbE, HbD, HbS, HbC ou HbConstantSpring est mesuré par (b) ci-dessus.

5. Procédé de mesure selon l'une quelconque des revendications 1 à 4, dans lequel au moins l'un des articles (1) à (5) suivants est satisfait :

(1) une pression osmotique de l'éluant L est de 35 mOsm à 200 mOsm ;
(2) une pression osmotique de l'éluant O est de 170 mOsm à 290 mOsm ;
(3) une pression osmotique de l'éluant M est de 160 mOsm à 210 mOsm ;
(4) un liquide mixte préparé en mélangeant l'éluant L et l'éluant O est utilisé et un rapport de mélange des éluants est le rapport de l'éluant L à l'éluant O qui se situe de 1:2 à 1:4 ; et
(5) un mélange mixte préparé en mélangeant l'éluant M et l'éluant L est utilisé et un rapport de mélange des éluants est le rapport de l'éluant M à l'éluant L qui est de 1:2 à 1:4.

6. Procédé de mesure selon l'une quelconque des revendications 1 à 5, dans lequel au moins l'un des articles (6) à (8) suivants est satisfait :

(6) un éluant A qui a un pH de 5,30 à 5,40 et une pression osmotique de 204 mOsm à 210 mOsm est en outre utilisé ;
(7) un éluant B qui un pH de 7,85 à 8,25 et une pression osmotique de 350 mOsm à 600 mOsm est en outre utilisé ; et
(8) un liquide mixte préparé en mélangeant un éluant B qui a un pH de 7,85 à 8,25 et une pression osmotique de 350 mOsm à 600 mOsm et un éluant A qui a un pH de 5,30 à 5,40 et une pression osmotique de 204 mOsm à 210 mOsm dans un rapport de mélange de l'éluant B à l'éluant A qui est de 1:2 à 1:4 est en outre utilisé.

7. Procédé de mesure selon la revendication 6, dans lequel au moins un variant d'hémoglobine de HbBart's, HbH, HbF, HbA1c, HbA0, HbA2, HbE, HbD, HbS, HbC ou HbConstantSpring est mesuré par (8) ci-dessus.

8. Appareil de mesure pour mesurer un variant d'hémoglobine par chromatographie en phase liquide de hautes performances, l'appareil de mesure comprenant :

une pompe d'alimentation pour fournir, seuls ou en combinaison un ou plusieurs éluants choisis dans le groupe constitué d'un éluant L qui contient un sel de métal dialcalin monohydrogéné d'acide phosphorique (composant 1) et un sel de métal monoalcalin dihydrogéné d'acide phosphorique (composant 2) comme composants et qui a une teneur en composant 1 de 0,08 % en masse à 0,50 % en masse par rapport à une masse totale de l'éluant L, une teneur en composant 2 de 0,04 % en masse à 1,2 % en masse par rapport à une masse totale de l'éluant L, et un rapport du composant 2 au composant 1 de 0,4 à 10, un éluant O qui a une teneur en composant 1 de moins de 0,07 % en masse par rapport à une masse totale de l'éluant O, une teneur en composant 2 de plus de 1,4 % en masse à 2,0 % en masse par rapport à une masse totale de l'éluant O, et un rapport du composant 2 au composant 1 de plus de 20, et un éluant M qui a une teneur en composant 1 de 0,40 % en masse à moins de 1,04 % en masse par rapport à une masse totale de l'éluant M, une teneur en composant 2 de plus de 0,26 % en masse à 0,88 % en masse par rapport à une masse totale de l'éluant M, et un rapport du composant 2 au composant 1 de plus de 0,25 à 2,2 ;
une colonne de séparation qui est raccordée à la pompe d'alimentation et à laquelle l'éluant est fourni ; et
une pluralité de réservoirs d'éluant stockant chacun de l'éluant L, de l'éluant O et de l'éluant M.

9. Appareil de mesure selon la revendication 8, dans lequel la pompe d'alimentation fournit des éluants selon l'un quelconque des articles (I) à (III) suivants :

(I) au moins un éluant choisi dans le groupe constitué de l'éluant L, de l'éluant O et de l'éluant M est fourni ou au moins deux éluants choisis dans le groupe constitué de l'éluant L, de l'éluant O et de l'éluant M sont fournis successivement sans mélange ;
(II) au moins un liquide mixte préparé en mélangeant les au moins deux éluants choisis dans le groupe constitué de l'éluant L, de l'éluant O et de l'éluant M est fourni ; et
(III) au moins un éluant choisi dans le groupe constitué de l'éluant L, de l'éluant O et de l'éluant M et au moins un liquide mixte préparé en mélangeant les au moins deux éluants choisis dans le groupe constitué de l'éluant L, de l'éluant O et de l'éluant M sont fournis successivement.

**10.** Appareil de mesure selon la revendication 8 ou la revendication 9, dans lequel, comme éluant, au moins l'un d'un éluant A qui a un pH de 5,30 à 5,40 et une pression osmotique de 204 mOsm à 210 mOsm ou d'un éluant B qui a un pH de 7,85 à 8,25 et une pression osmotique de 350 mOsm à 600 mOsm est en outre fourni, ou un liquide mixte préparé en mélangeant l'éluant A et l'éluant B est en outre fourni.

**11.** Appareil de mesure selon l'une quelconque des revendications 8 à 10, dans lequel l'appareil a une seule pompe d'alimentation.

**12.** Eluant selon l'un quelconque des articles (1) à (3) suivants :

(1) un éluant L convenant pour mesurer au moins un type d'hémoglobine choisi parmi une hémoglobine anormale ou une hémoglobine qui est un marqueur pour la thalassémie par chromatographie en phase liquide de hautes performances, l'éluant comprenant :

un sel de métal dialcalin monohydrogéné d'acide phosphorique (composant 1) ; et
un sel de métal monoalcalin dihydrogéné d'acide phosphorique (composant 2), dans lequel :

une teneur du composant 1 est de 0,08 % en masse à 0,50 % en masse par rapport à une masse totale de l'éluant L,
une teneur en composant 2 est de 0,04 % en masse à 1,2 % en masse par rapport à une masse totale de l'éluant L et
un rapport du composant 2 au composant 1 est de 0,4 à 10 ;

(2) un éluant O convenant pour la mesure d'au moins un type d'hémoglobine choisi parmi une hémoglobine anormale ou une hémoglobine qui est un marqueur pour la thalassémie par chromatographie en phase liquide de hautes performances, l'éluant comprenant :

un sel de métal dialcalin monohydrogéné d'acide phosphorique (composant 1) ; et
un sel de métal monoalcalin dihydrogéné d'acide phosphorique (composant 2), dans lequel :

une teneur en composant 1 est inférieure à 0,07 % en masse par rapport à une masse totale de l'éluant O,
une teneur en composant 2 est de plus de 1,4 % en masse à 2,0 % en masse par rapport à une masse totale de l'éluant O et
un rapport du composant 2 au composant 1 est supérieur à 20 ; et

(3) un éluant M convenant pour la mesure d'au moins un type d'hémoglobine choisi parmi une hémoglobine anormale ou une hémoglobine qui est un marqueur pour la thalassémie par chromatographie en phase liquide de hautes performances, l'éluant comprenant :

un sel de métal dialcalin monohydrogéné d'acide phosphorique (composant 1) ; et
un sel de métal monoalcalin dihydrogéné d'acide phosphorique (composant 2), dans lequel :

une teneur en composant 1 est de 0,40 % en masse à moins de 1,04 % en masse par rapport à une masse totale de l'éluant M,
une teneur en composant 2 est de plus de 0,26 % en masse à 0,88 % en masse par rapport à une masse totale de l'éluant M et
un rapport du composant 2 au composant 1 est de plus de 0,25 à 2,2.

13. Combinaison d'éluants convenant pour la mesure d'au moins un type d'hémoglobine choisi parmi une hémoglobine ou une hémoglobine qui est un marqueur pour la thalassémie par chromatographie en phase liquide à hautes performances, la combinaison d'éluants comprenant :

au moins deux éluants choisis dans le groupe constitué d'un éluant L qui contient un sel de métal dialcalin monohydrogéné d'acide phosphorique (composant 1) et un sel de métal monoalcalin dihydrogéné d'acide phosphorique (composant 2) comme composants et qui a une teneur en composant 1 de 0,08 % en masse à 0,50 % en masse par rapport à une masse totale de l'éluant L, une teneur en composant 2 de 0,04 % en masse à 1,2 % en masse par rapport à une masse totale de l'éluant L et un rapport du composant 2 au composant 1 de 0,4 à 10 ; un éluant O qui a une teneur en composant 1 de moins de 0,07 % en masse par rapport à une masse totale de l'éluant O, une teneur en composant 2 de plus de 1,4 % en masse à 2,0 % en masse par rapport à une masse totale de l'éluant O, et un rapport du composant 2 au composant 1 de plus de 20 ; et un éluant M qui a une teneur en composant 1 de 0,40 % en masse à moins de 1,04 % en masse par rapport à une masse totale de l'éluant M, une teneur en composant de plus de 0,26 % en masse à 0,88 % en masse par rapport à une masse totale de l'éluant M et un rapport du composant 2 au composant 1 de plus de 0,25 à 2,2.

# FIG.1

FIG.2

FIG.3

FIG.4

# FIG.5

MEASUREMENT TIME(SECONDS)

# FIG.6

# FIG.7

EP 2 960 648 B1

MEASUREMENT TIME(SECONDS)

## FIG.8

## FIG.9

MEASUREMENT TIME(SECONDS)

EP 2 960 648 B1

EP 2 960 648 B1

# FIG.10

| | |
|---|---|
| —— | HbS, HbC |
| ----- | Hbconstant spring |

HbS

HbC

Hbconstant spring

0   20   40   60   80   100   120   140   160   180

MEASUREMENT TIME(SECONDS)

# FIG.11

MEASUREMENT TIME(SECONDS)

EP 2 960 648 B1

EP 2 960 648 B1

# FIG.12

LIQUID L(OSMOTIC PRESSURE vs pH)

FIG.13

LIQUID L(OSMOTIC PRESSURE vs COMPONENT 2/COMPONENT 1)

EP 2 960 648 B1

# FIG.14

LIQUID M(OSMOTIC PRESSURE vs pH)

FIG.15

LIQUID M(OSMOTIC PRESSURE vs COMPONENT 2/COMPONENT 1)

COMPONENT 2/COMPONENT 1

OSMOTIC PRESSURE(mOsm)

FIG.16

LIQUID O (OSMOTIC PRESSURE vs pH)

OSMOTIC PRESSURE(mOsm)

EP 2 960 648 B1

# FIG.17

LIQUID O (OSMOTIC PRESSURE vs COMPONENT 2/COMPONENT 1)

EP 2 960 648 B1

# FIG.18

LIQUID B' (OSMOTIC PRESSURE vs pH)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP H05281222 A
- JP 2009236768 A
- EP 2562539 A1
- US 20090071233 A1